(19) Europäisches Patentamt / European Patent Office / Office européen des brevets

(11) **EP 4 559 922 A1**

(12) **EUROPEAN PATENT APPLICATION**
published in accordance with Art. 153(4) EPC

(43) Date of publication:
**28.05.2025 Bulletin 2025/22**

(21) Application number: **23841716.6**

(22) Date of filing: **13.02.2023**

(51) International Patent Classification (IPC):
***C07F 9/6558*** (2006.01) ***C07C 213/08*** (2006.01)
***C07C 215/40*** (2006.01) ***C07C 277/08*** (2006.01)
***A61K 31/675*** (2006.01) ***A61P 37/08*** (2006.01)

(52) Cooperative Patent Classification (CPC):
**A61K 31/675; A61P 37/08; C07C 213/08; C07C 215/40; C07C 277/08; C07C 279/14; C07F 9/6558**

(86) International application number:
**PCT/CN2023/075588**

(87) International publication number:
**WO 2024/016638 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.07.2022 CN 202210875404**

(71) Applicant: **HC Synthetic Pharmaceutical Co., Ltd.**
**Xi’an, Shaanxi 710000 (CN)**

(72) Inventors:
- **YANG, Cheng**
  **Xi’an, Shaanxi 710000 (CN)**
- **ZHANG, Qiyuan**
  **Xi’an, Shaanxi 710000 (CN)**

(74) Representative: **Patentwerk B.V.**
**P.O. Box 1514**
**5200 BN ’s-Hertogenbosch (NL)**

(54) **ANTIHISTAMINE COMPOUND, PREPARATION METHOD THEREFOR AND USE THEREOF**

(57) Provided are a series of antihistaminic compounds of Formula I, pharmaceutically acceptable salts thereof, and a preparation method thereof, the the use of the antihistaminic compounds of Formula I and the pharmaceutically acceptable salts in treating diseases such as seasonal and perennial allergic rhinitis, allergic conjunctivitis, and urticaria.

EP 4 559 922 A1

## Description

This application claims the priority to the Chinese patent application No. 202210875404.X filed with the China National Intellectual Property Administration on July 21, 2022, titled "Antihistaminic compounds, preparation method and use thereof", the entire content of which is incorporated by reference into the present disclosure.

## TECHNICAL FIELD

The present disclosure belongs to the technical field of pharmaceutical technology, and more specifically, relates to a series of antihistaminic compounds, a preparation method thereof, and use thereof in medical field.

## BACKGROUND

Allergic diseases are major diseases that affect human health. Developing and obtaining anti-allergic drugs with stronger efficacy and minor adverse reactions have always been one of the research hotspots of pharmaceutical professionals around the world. Hi receptor antagonists are main drugs for clinical treatment of allergic diseases. Studies on structure-activity relationship have shown that Hi receptor antagonists are generally composed of an aromatic ring region, a connecting segment, and a basic amine region. Based on the structural types, Hi receptor antagonists can be roughly divided into ethylenediamines, aminoalkyl ethers, propylamines, and tricyclic drugs. The main adverse reactions of Hi receptor antagonists in clinical applications are central nervous system inhibition and cardiac toxicity. The former adverse reaction is due to high lipid solubility of Hi receptor antagonist molecules which can easily pass through the blood-cerebrospinal fluid barrier, thus producing a sedative and hypnotic effect. The latter adverse reaction is due to the ability of some Hi receptor antagonists to inhibit delayed rectifier potassium current and potassium ion channels on human cardiomyocytes, leading to prolongation of QT interval of the electrocardiogram, while inducing torsades de pointes (TdP) and triggering fatal arrhythmias. Among them, the $H_1$ receptor antagonists, astemizole and terferadine, have been withdrawn from the market due to cardiotoxicity concerns.

Many tricyclic antihistamine derivatives (including highly active ketolifen) have been synthesized. However, as the research deepens, its side effects gradually emerge. The most significant side effect of ketotifen fumarate is the central nervous system depressant effect such as drowsiness or fatigue. This side effect becomes more obvious in adults and is one of the main reasons for its limited clinical use. The drowsiness or fatigue can reduce the quality of life of asthma patients and child asthma patients. Therefore, it is of great research value to modify the structure of ketotifen and screen for new drugs with stronger antihistamine activity, better physicochemical properties, and higher stability.

## SUMMARY OF THE INVENTION

An objective of the present disclosure is to provide antihistaminic compounds and pharmaceutically acceptable salts thereof.

Another objective of the present disclosure is to provide a method for preparing these compounds.

A yet another objective of the present disclosure is to provide a medical use of the compounds. Compounds of this type generally have a therapeutic effect in treating diseases such as seasonal and perennial allergic rhinitis, allergic conjunctivitis, and urticaria.

A still yet another objective of the present disclosure is to disclose a pharmaceutical composition comprising the compounds and pharmaceutically acceptable salt thereof as a main active ingredient.

The present disclosure will be described in detail in combination with the objectives of the present disclosure.

Specifically, the present disclosure relates to a compound having Formula I and a pharmaceutically acceptable salt thereof:

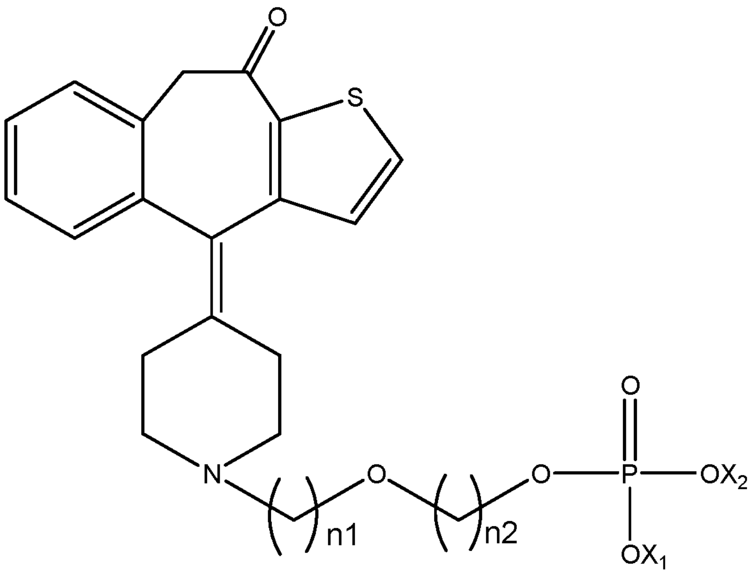

I

wherein $X_1$ is H, an alkali metal, an amino acid, meglumine, choline; $X_2$ is H, an alkali metal, an amino acid, meglumine, choline, n1 is an integer of 1-5, and n2 is an integer of 1-3.

In the compounds of the present disclosure, the thiophene group introduced into the compound structure can improve the activity of the compound, thereby improving the efficacy. In addition, the structure of the phosphate group can increase water solubility, and the phosphate bond can be hydrolyzed in the body via the action of phosphatase, thereby promoting drug metabolism.

The present disclosure provides a compound of Formula I and a pharmaceutically acceptable salt thereof, wherein the compound is selected from the group consisting of:

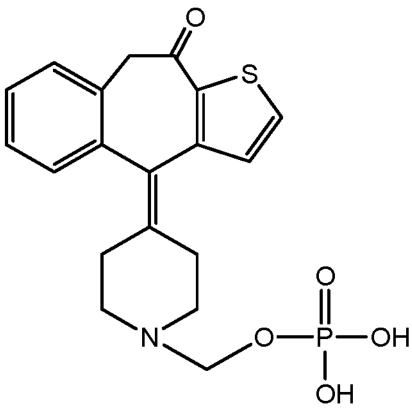

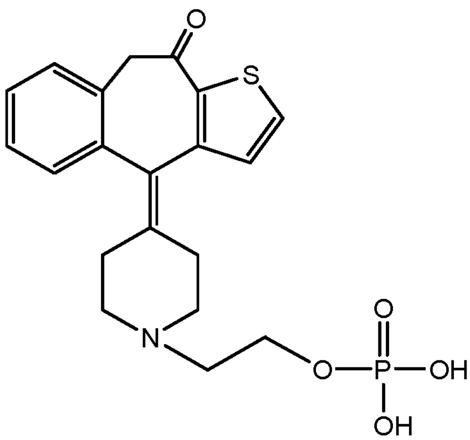

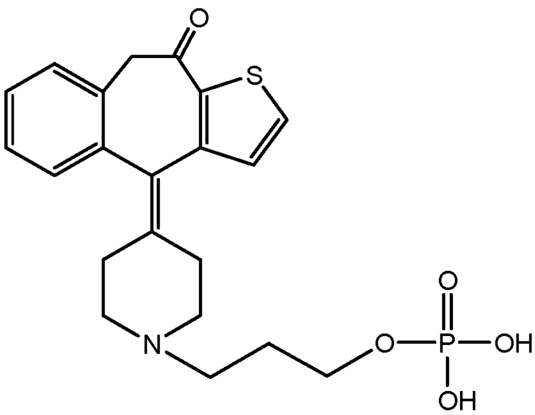

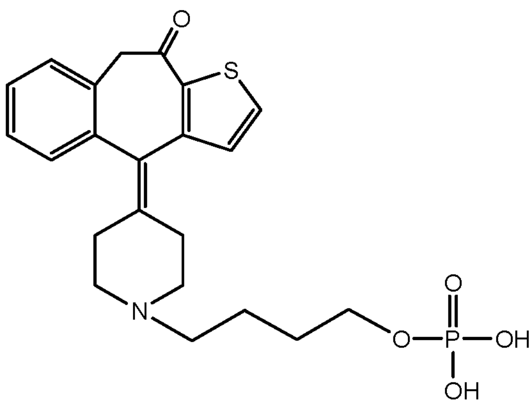

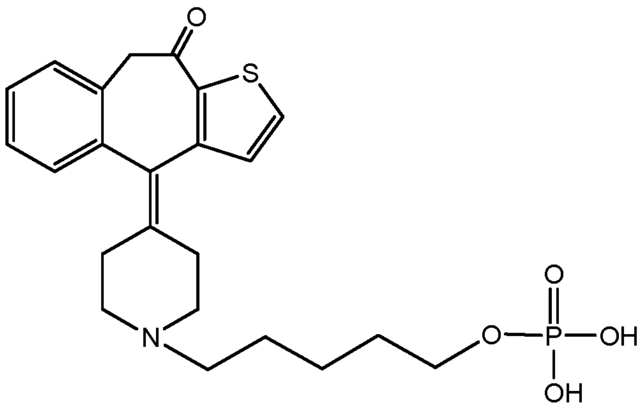

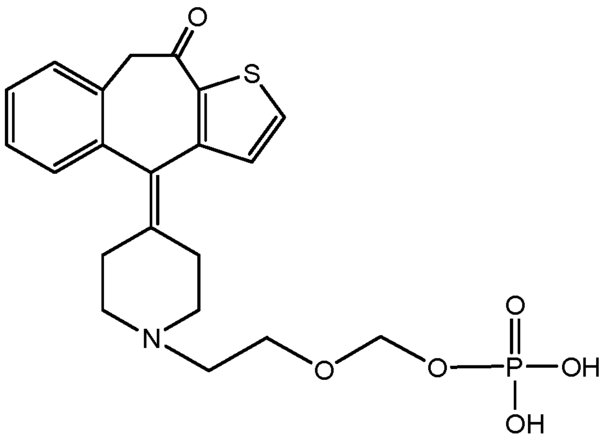

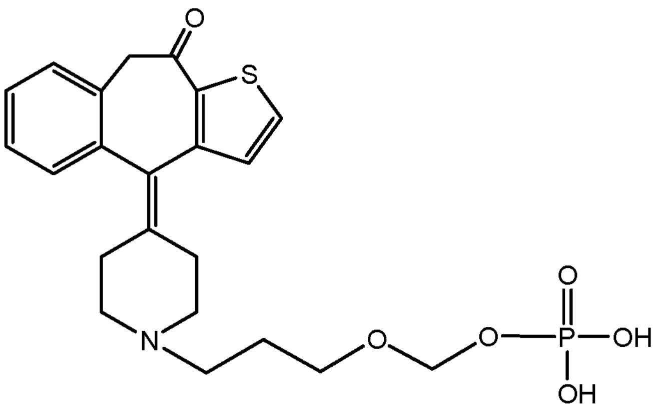

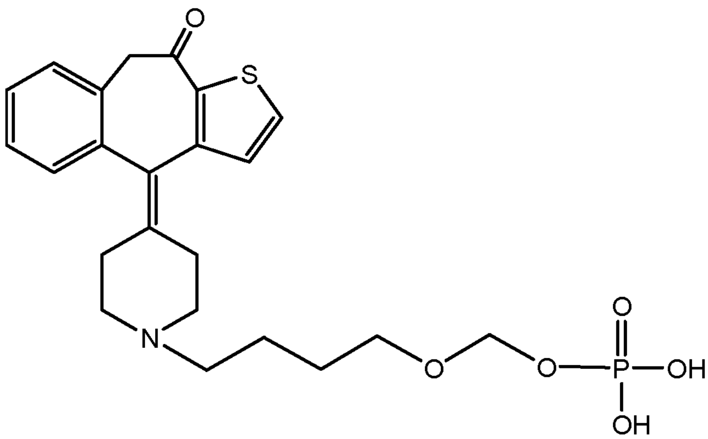

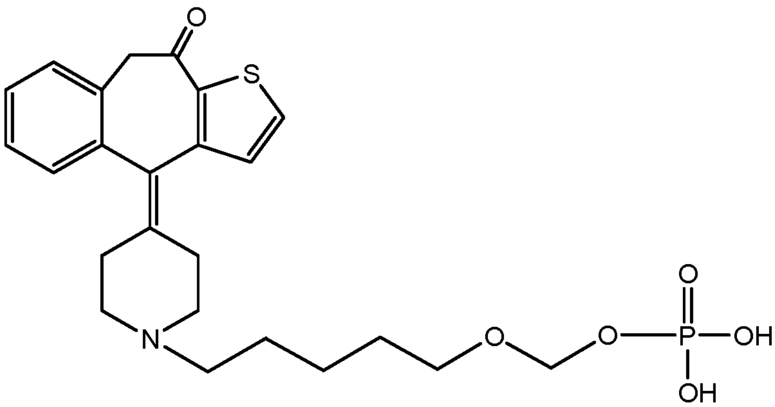

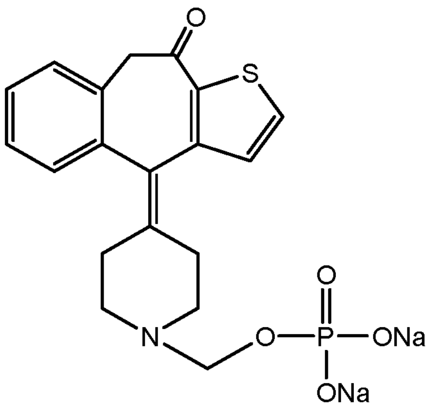

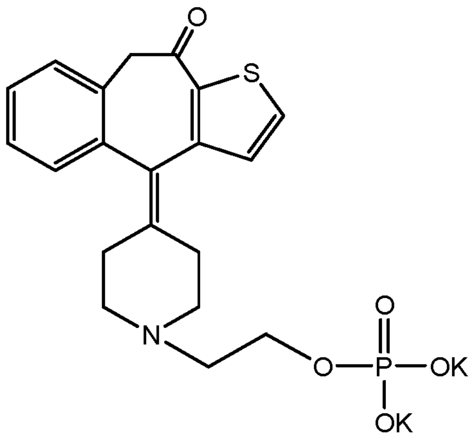

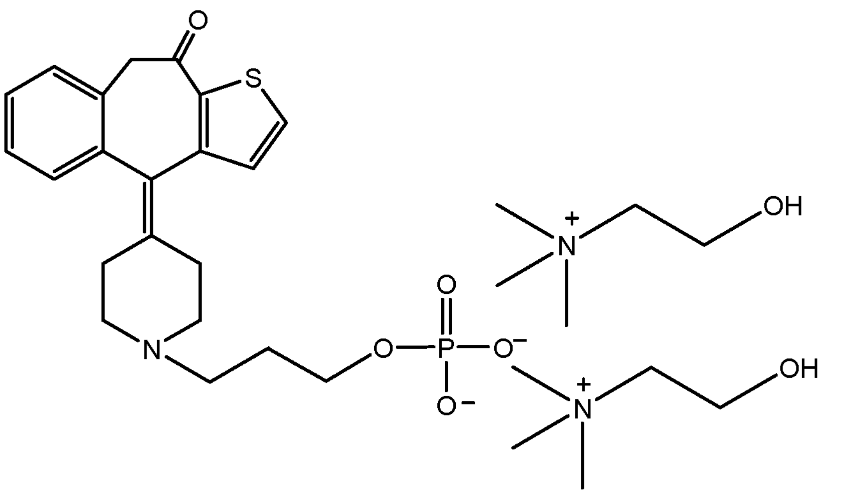

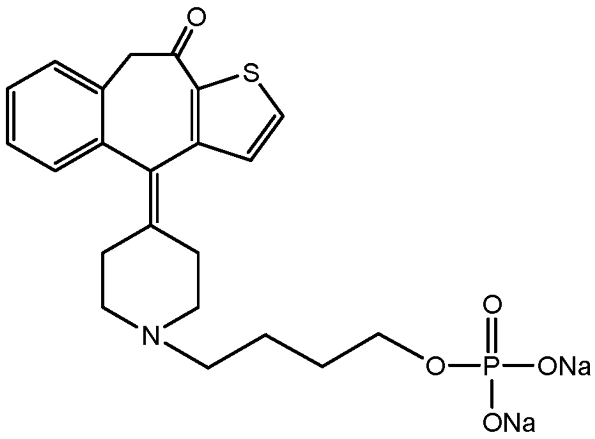

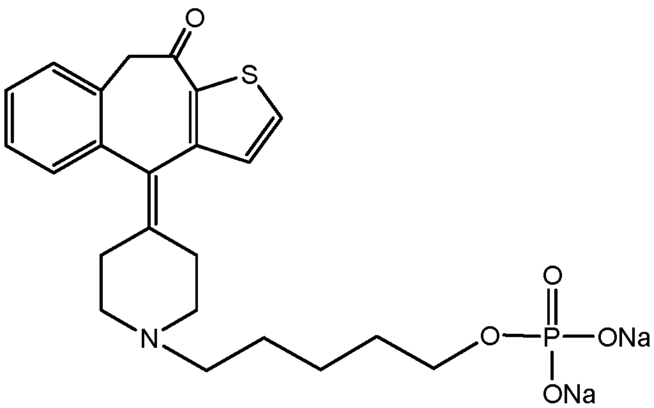

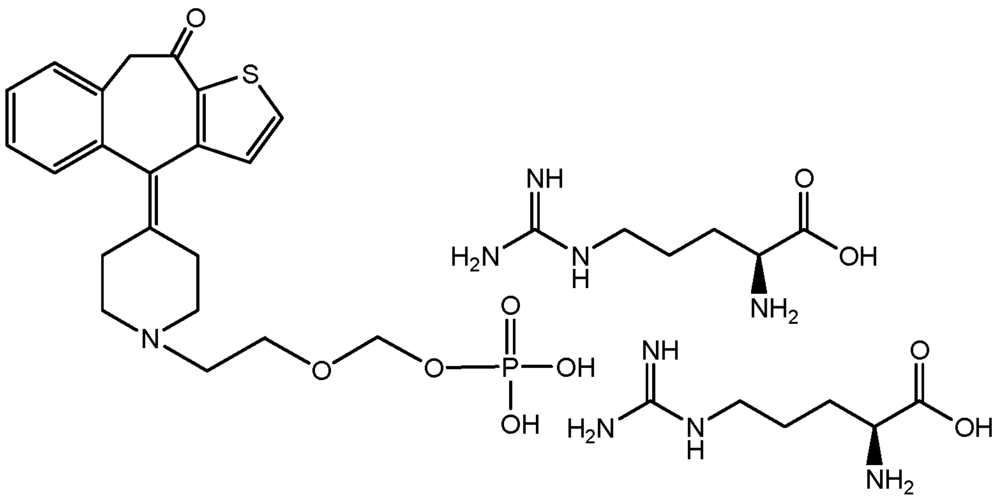

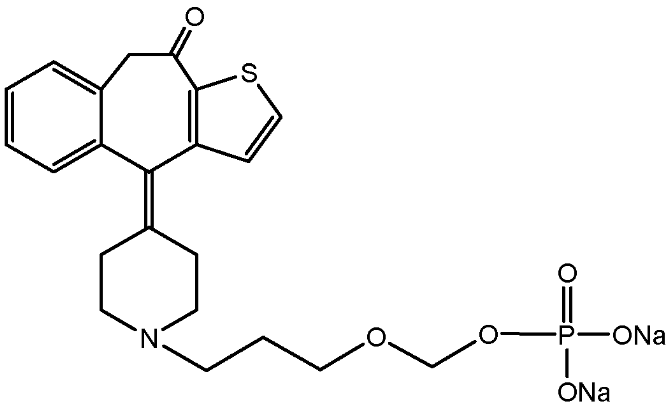

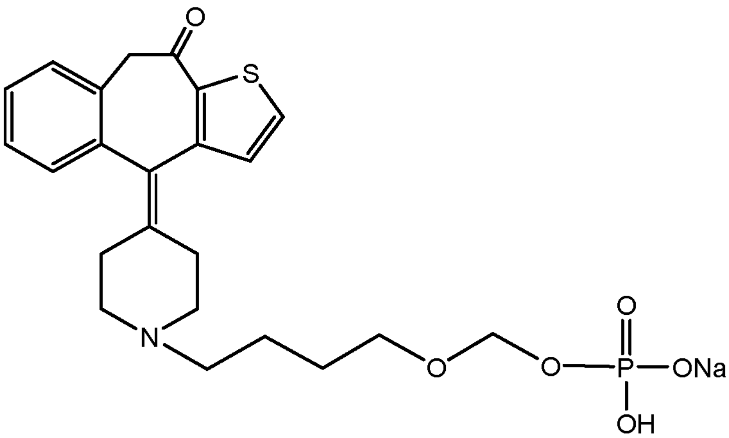

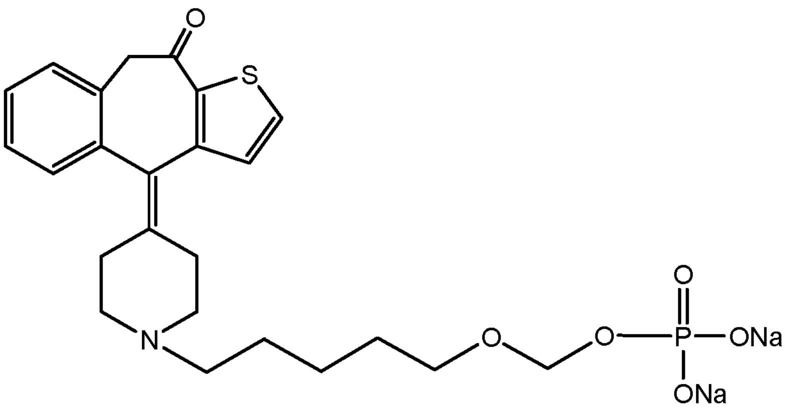

.

The present disclosure also provides the use of the compound of Formula I, the pharmaceutically acceptable salt thereof, or a pharmaceutically acceptable composition comprising the same in the treatment of seasonal and perennial allergic rhinitis, allergic conjunctivitis, and urticaria.

The compound of Formula I is prepared by a method as follows:

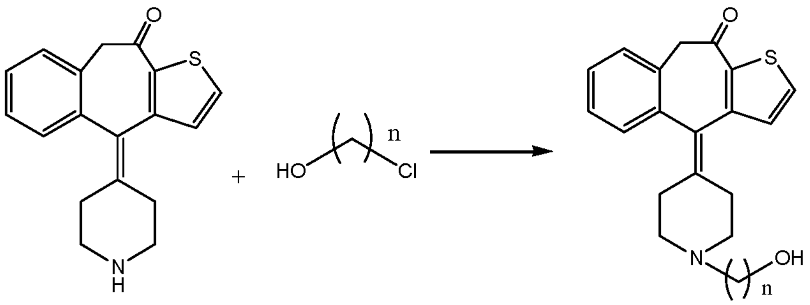

M1 I

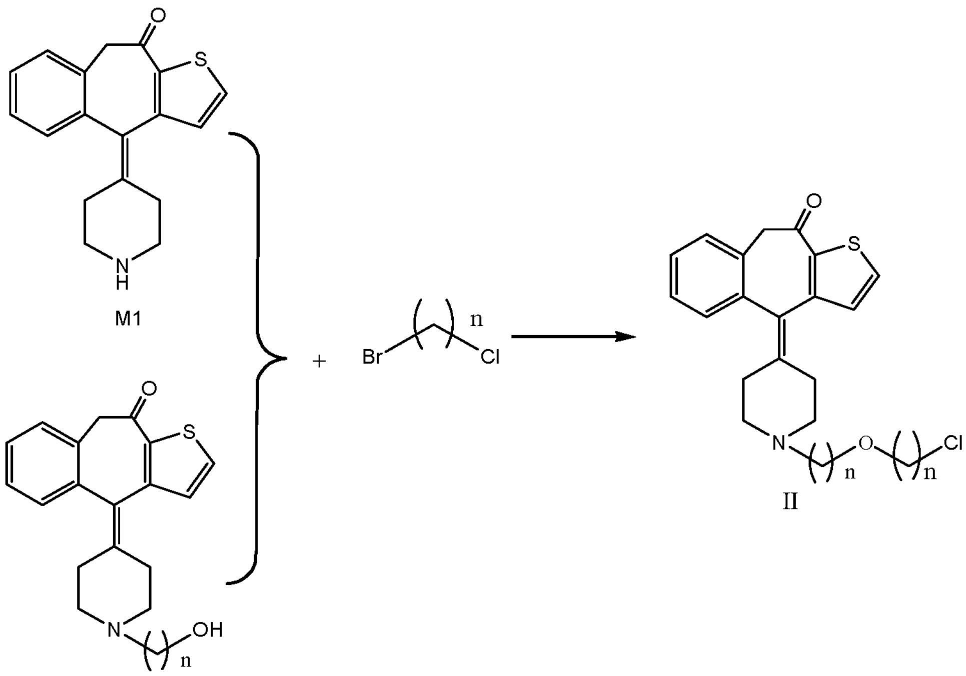

M1

II

I

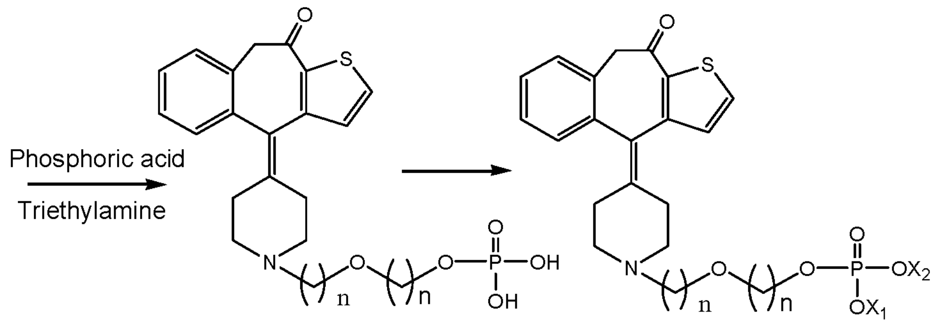


III IV

wherein $X_1$, $X_2$, and n are defined as above.

Among them, the synthesis of Compound M1 has been reported in various literatures, and it can be prepared by those skilled in the art by referring to those reported methods.

Compound M1 is dissolved in a non-protonic solvent such as dichloromethane, chloroform, acetone, acetonitrile, tetrahydrofuran, N,N-dimethylformamide (DMF), pyridine, or toluene, and a solution of a halogenated alcohol or a solution of a corresponding organic solvent thereof is added dropwise. An organic base or an inorganic base such as triethylamine, pyridine, potassium tert-butoxide, sodium methoxide, sodium ethoxide, potassium carbonate, sodium carbonate, potassium bicarbonate, sodium bicarbonate, sodium hydroxide, or potassium hydroxide is used as an acid acceptor, and the reaction is carried out at -5°C to 60°C to obtain Compound I. The molar ratio of M1 to halogenated alcohol is 1:(1-10).

Compound M1 or Compound I is dissolved in a non-protonic solvent such as dichloromethane, chloroform, acetone, acetonitrile, tetrahydrofuran, N,N-dimethylformamide (DMF), pyridine, or toluene, and a solution of a halogenated alcohol or a solution of a corresponding organic solvent thereof is added dropwise. An organic base or an inorganic base such as triethylamine, pyridine, potassium tert-butoxide, sodium methoxide, sodium ethoxide, potassium carbonate, sodium carbonate, potassium bicarbonate, sodium bicarbonate, sodium hydroxide, or potassium hydroxide is used as an acid acceptor, and the reaction is carried out at -5°C to 60°C to obtain Compound II. The molar ratio of M1 or Compound I to chlorobromoalkane is 1:(1-10).

The resulting product Compound II is dissolved in acetonitrile, acetone, methanol, ethanol, or tetrahydrofuran, triethylamine is added and stirred evenly, phosphoric acid is added dropwise, and the reaction is carried out at 25°C to 80°C to obtain Compound III.

The resulting product Compound III is dissolved in acetonitrile, acetone, methanol, ethanol, or tetrahydrofuran, and sodium hydroxide, potassium hydroxide, choline hydroxide, arginine, or proline is added. The reaction is carried out at 25°C to 80°C to obtain Compound IV, i.e., the pharmaceutically acceptable salt.

The compounds of the present disclosure are used in the form of pharmaceutical preparations, and the administration route may be a parenteral route (such as intravenous, intramuscular) or an oral route.

The pharmaceutical composition of the present disclosure compound is prepared as follows: conjugating the compound of the present disclosure with a pharmaceutically acceptable solid or liquid carrier, and optionally with a pharmaceutically acceptable adjuvant and an excipient using a standard technique and a conventional technique to prepare microparticles or microspheres. Solid dosage forms include tablets, dispersible granules, capsules, sustained-release tablets, sustained-release pellets, and etc. The solid carrier can be at least one substance which can act as a diluent, a flavoring agent, a solubilizer, a lubricant, a suspending agent, a binder, a disintegrating agent, and an encapsulating agent. Inert solid carriers include magnesium phosphate, magnesium stearate, talcum powder, lactose, pectin, propylene glycol, polysorbate 80, dextrin, starch, gelatin, cellulose materials (e.g., methyl cellulose and microcrystalline cellulose), low melting point paraffin, polyethylene glycol, mannitol, cocoa butter and the like. Liquid dosage forms include solutions, suspensions such as injections, powders, and the like.

The amount of active ingredient (the compound of the present disclosure) contained in the pharmaceutical composition and the unit dosage form can be specifically administered based on the patient's condition and the doctor's diagnosis. The amount or concentration of the compound can be adjusted within a wide range. Generally, the amount of the active compound ranges from 0.5 wt% to 90 wt% of the composition. A preferred range is 0.5% to 70%.

## DETAILED DESCRIPTION OF THE EMBODIMENTS

The present disclosure is further described below in conjunction with specific embodiments, but the present disclosure is not limited thereto.

Preparation Example

Example 1: Preparation of Intermediate 1

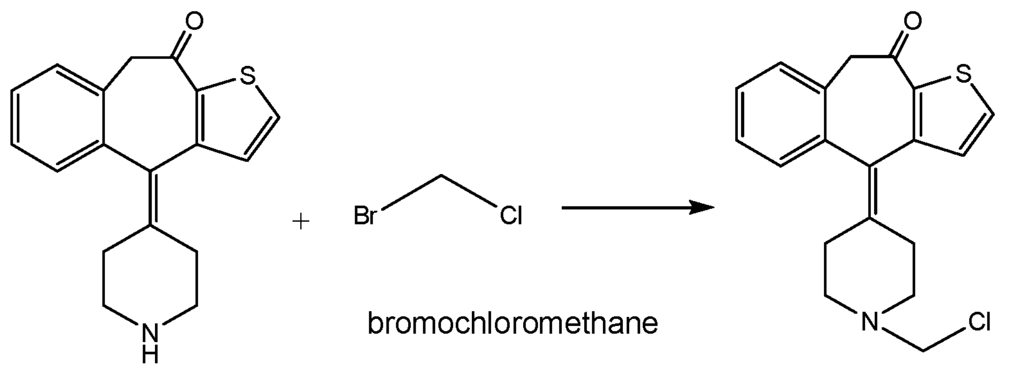

M1

bromochloromethane

Intermediate 1

To a reaction flask were added 100ml of tetrahydrofuran, 10g of Compound M1, 43.80g of bromochloromethane, and 3.38g of sodium hydroxide, and the temperature of the mixture was slowly raised to 60°C. The reaction was conducted for 6h under stirring. After the reaction was completed, the reaction solution was filtered, and the filtrate was evaporated

under reduced pressure to remove the solvents to obtain 9.11g of Intermediate 1 with a yield of 78.3%.

Example 2: Preparation of Intermediate 2

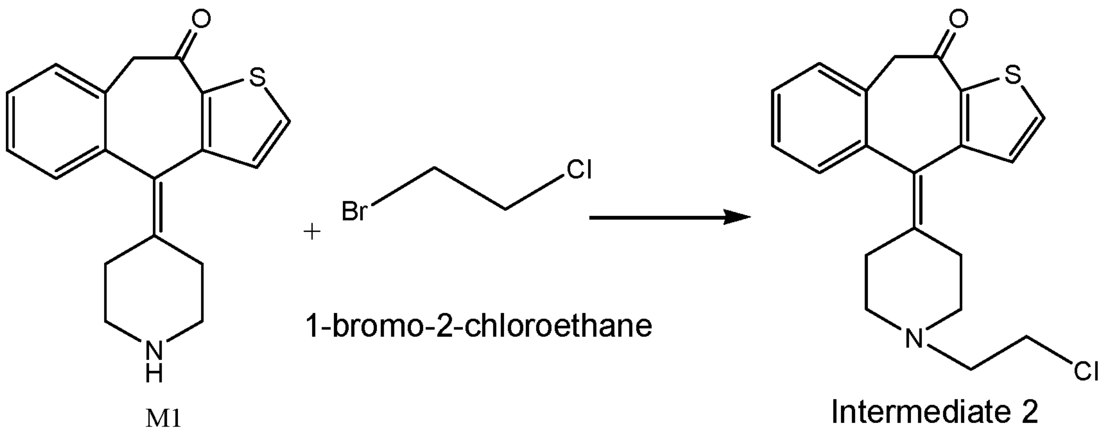


Intermediate 2 was prepared using the method for preparing Intermediate 1 in Example 1, with bromochloromethane replaced with 1-bromo-2-chloroethane, and other materials remained unchanged.

Example 3: Preparation of Intermediate 3

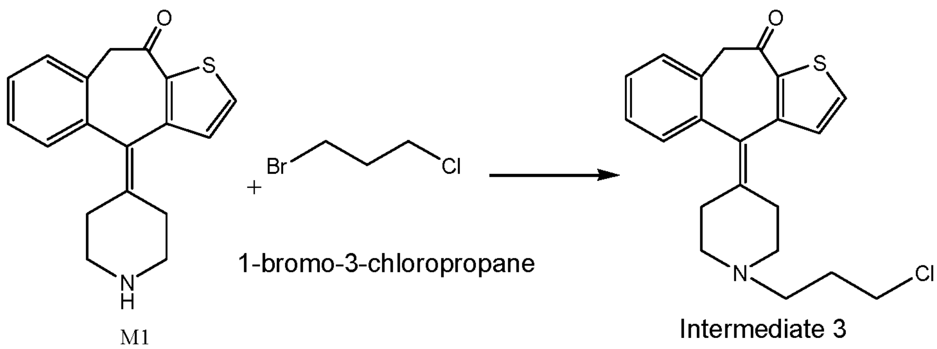


Intermediate 3 was prepared using the method for preparing Intermediate 1 in Example 1, with bromochloromethane replaced with 1-bromo-3-chloropropane, and other materials remained unchanged.

Example 4: Preparation of Intermediate 4

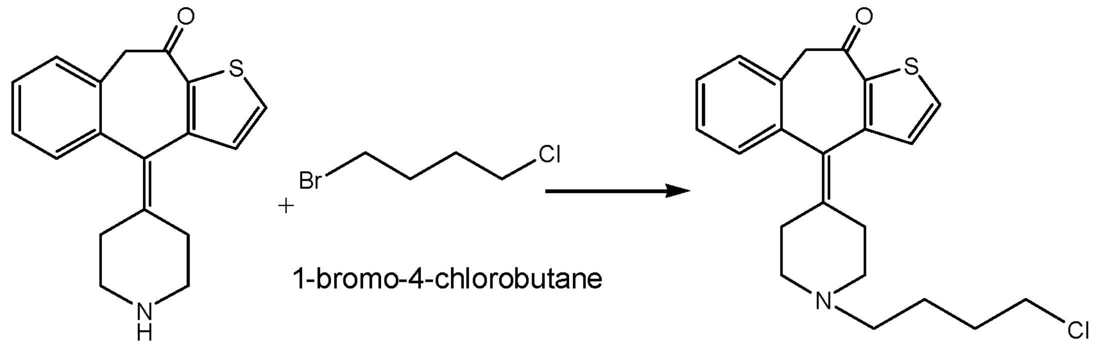


M1 Intermediate 4

Intermediate 4 was prepared using the method for preparing Intermediate 1 in Example 1, with bromochloromethane replaced with 1-bromo-4-chlorobutane, and other materials remained unchanged.

Example 5: Preparation of Intermediate 5

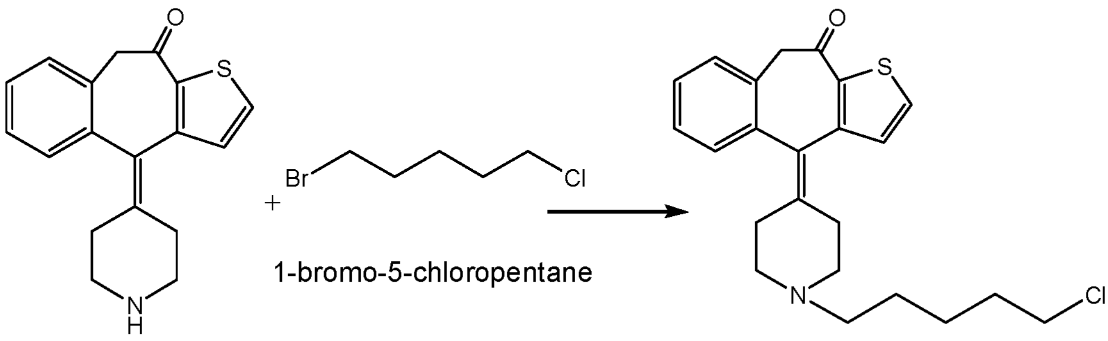


M1 Intermediate 5

Intermediate 5 was prepared using the method for preparing Intermediate 1 in Example 1, with bromochloromethane replaced with 1-bromo-5-chloropentane, and other materials remained unchanged.

Example 6: Preparation of Intermediate 6

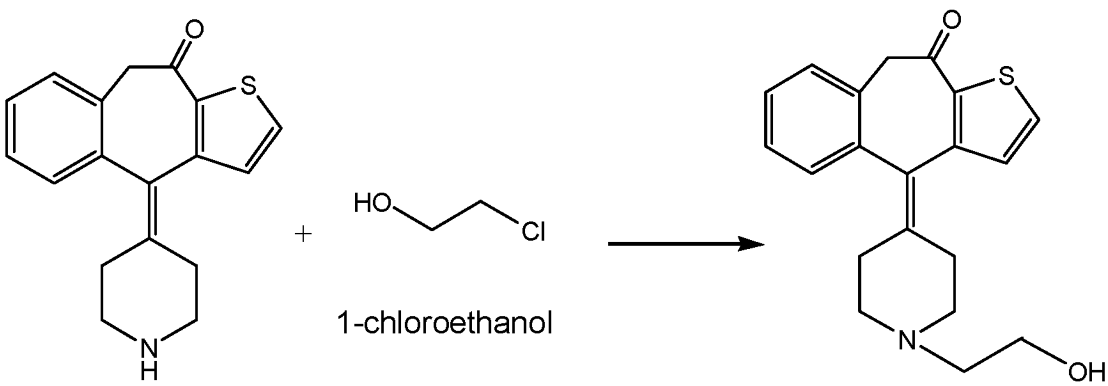


M1 Intermediate 6

Intermediate 6 was prepared using the method for preparing Intermediate 1 in Example 1, with bromochloromethane replaced with 1-chloroethanol, and other materials remained unchanged.

Example 7: Preparation of Intermediate 7

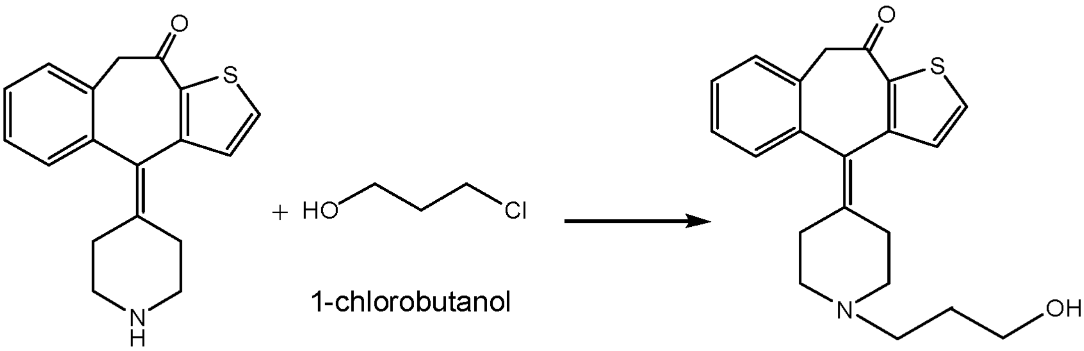

1-chlorobutanol

M1

Intermediate 7

Intermediate 7 was prepared using the method for preparing Intermediate 1 in Example 1, with bromochloromethane replaced with 1-chloropropanol, and other materials remained unchanged.

Example 8: Preparation of Intermediate 8

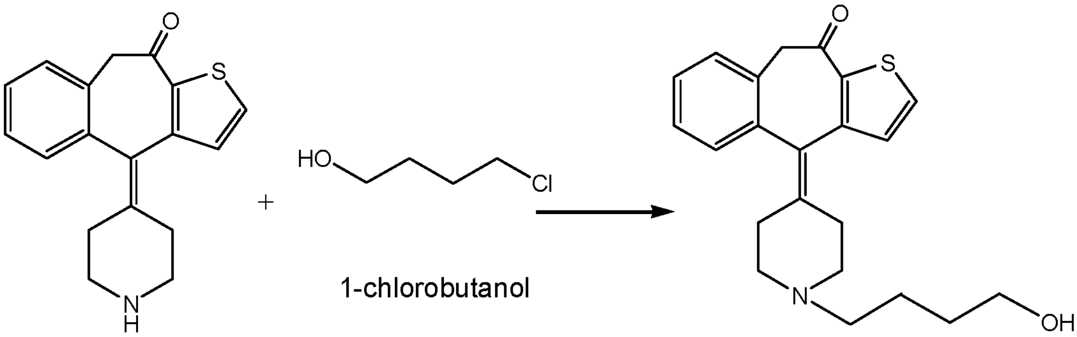

1-chlorobutanol

M1

Intermediate 8

Intermediate 8 was prepared using the method for preparing Intermediate 1 in Example 1, with bromochloromethane replaced with 1-chlorobutanol, and other materials remained unchanged.

Example 9: Preparation of Intermediate 9

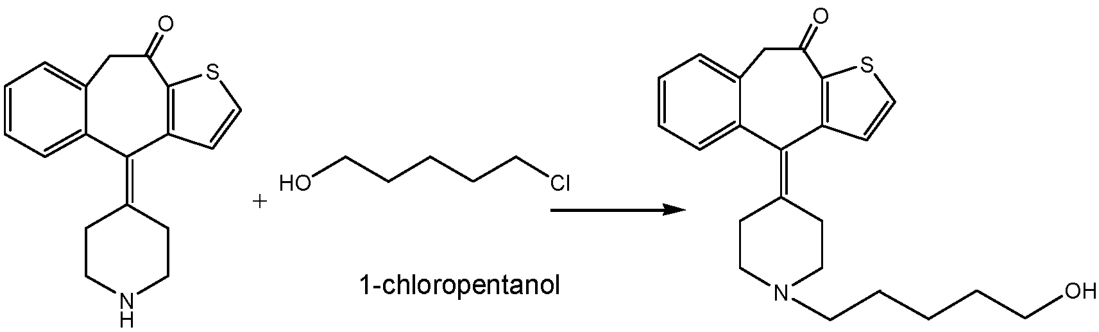

1-chloropentanol

M1

Intermediate 9

Intermediate 9 was prepared using the method for preparing Intermediate 1 in Example 1, with bromochloromethane replaced with 1-chloropentanol, and other materials remained unchanged.

Example 10: Preparation of Intermediate 10

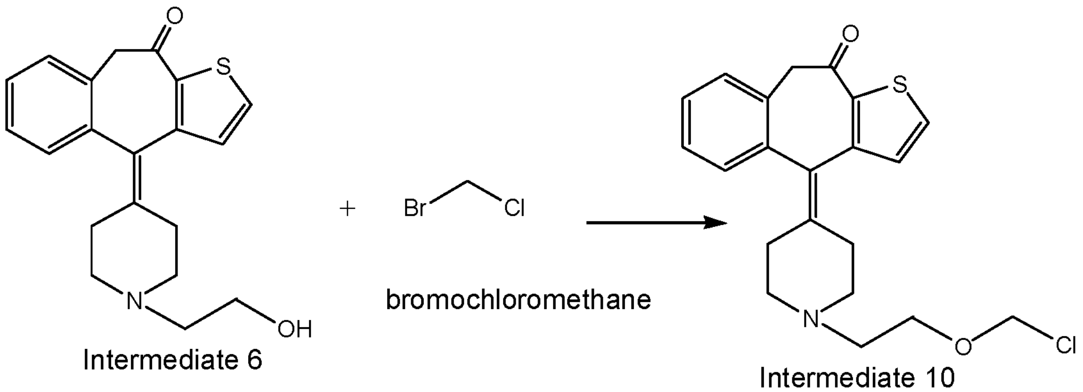

bromochloromethane

Intermediate 6

Intermediate 10

Intermediate 10 was prepared using the method for preparing Intermediate 1 in Example 1, with M1 replaced with Intermediate 6, and other materials remained unchanged.

Example 11: Preparation of Intermediate 11

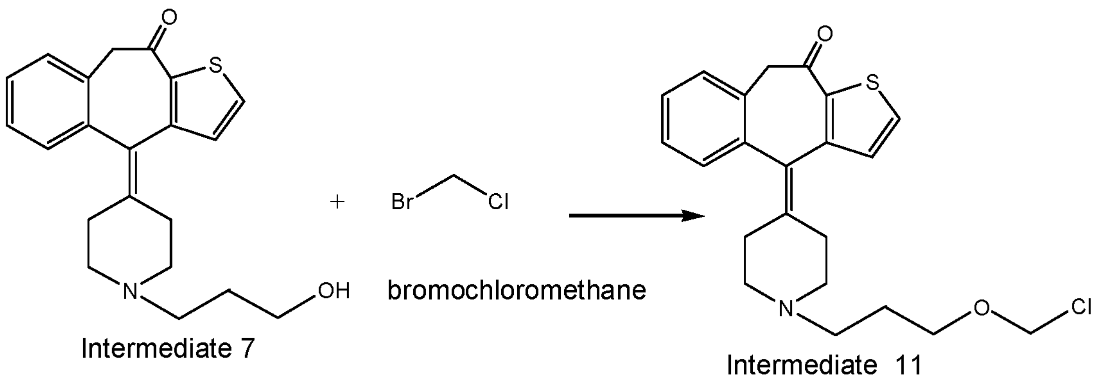

bromochloromethane

Intermediate 7

Intermediate 11

Intermediate 11 was prepared using the method for preparing Intermediate 1 in Example 1, with M1 replaced with Intermediate 7, and other materials remained unchanged.

Example 12: Preparation of Intermediate 12

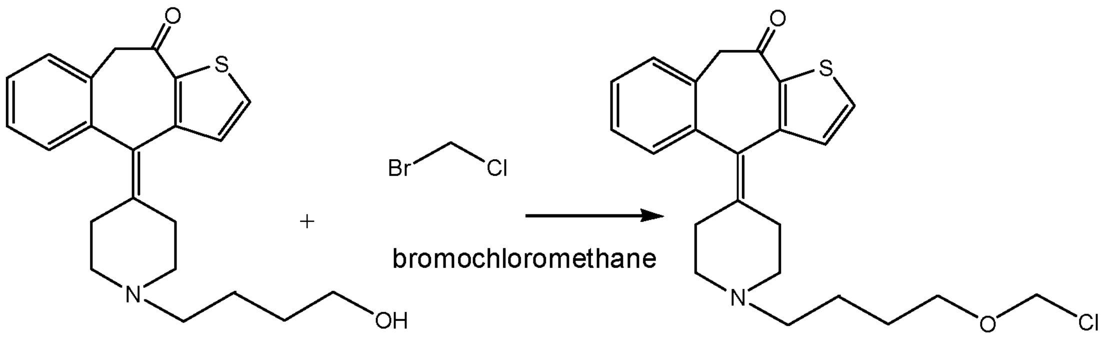


Intermediate 8 Intermediate 12

Intermediate 12 was prepared using the method for preparing Intermediate 1 in Example 1, with M1 replaced with Intermediate 8, and other materials remained unchanged.

Example 13: Preparation of Intermediate 13

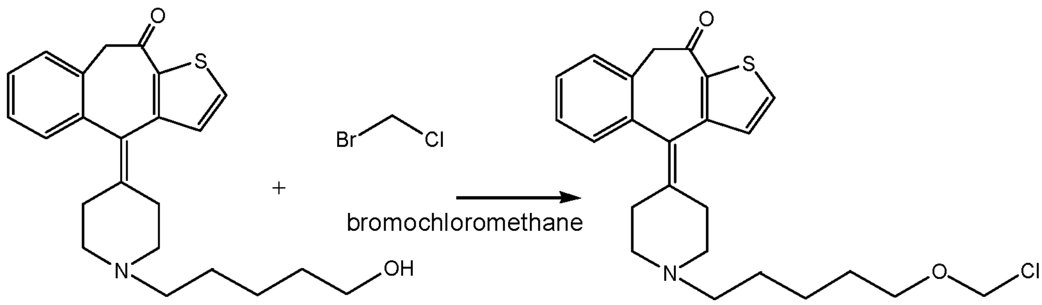


Intermediate 9 Intermediate 13

Intermediate 13 was prepared using the method for preparing Intermediate 1 in Example 1, with M1 replaced with Intermediate 9, and other materials remained unchanged.

Example 14: Preparation of Compound III-1

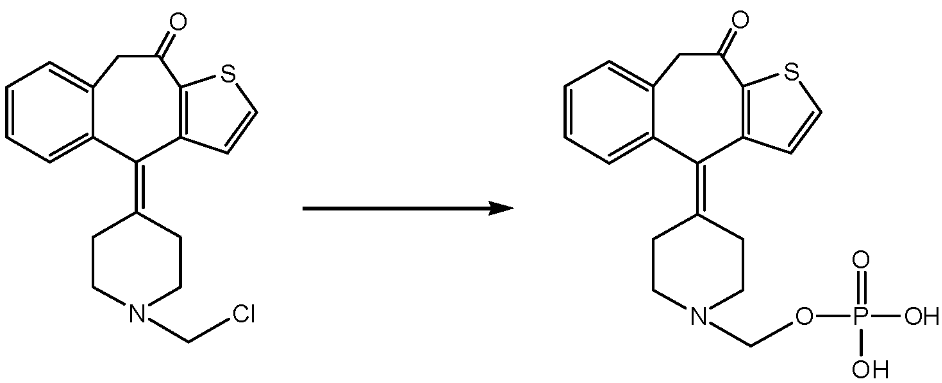

bromochloromethane 1 III-1

To a reaction flask, 30ml of acetonitrile was placed, and 7.34g of triethylamine and 8.38g of phosphoric acid were added. The resulting mixture was heated to 60°C under stirring to dissolve the solids. Then 10g of Intermediate 1 was slowly added, and the temperature was held at 60°C for 6 hours after the addition. The reaction was ended and the solvent was removed by evaporation under reduced pressure at 60°C. After evaporation to dryness, 20ml of water was added and stirred, and concentrated hydrochloric acid was slowly added dropwise to adjust pH to 1.5. Fifty milliliters (25 ml × 2) of

ethyl acetate was added to extract the aqueous layer, the aqueous layer was removed, and then ethyl acetate was removed by evaporation at 50°C under reduced pressure to obtain 9.1g of Compound III-1 with a yield of 77.1%.

Example 15: Preparation of Compound III-2

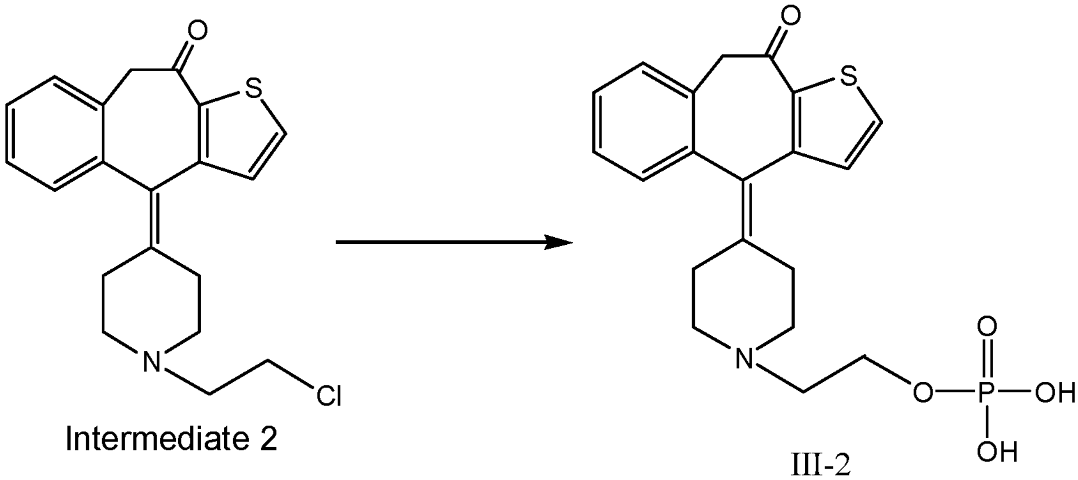

Intermediate 2

III-2

Compound III-2 was prepared using the method for preparing Intermediate III-1 in Example 14, with Intermediate 1 replaced with Intermediate 2, and other materials remained unchanged.

Example 16: Preparation of Compound III-3

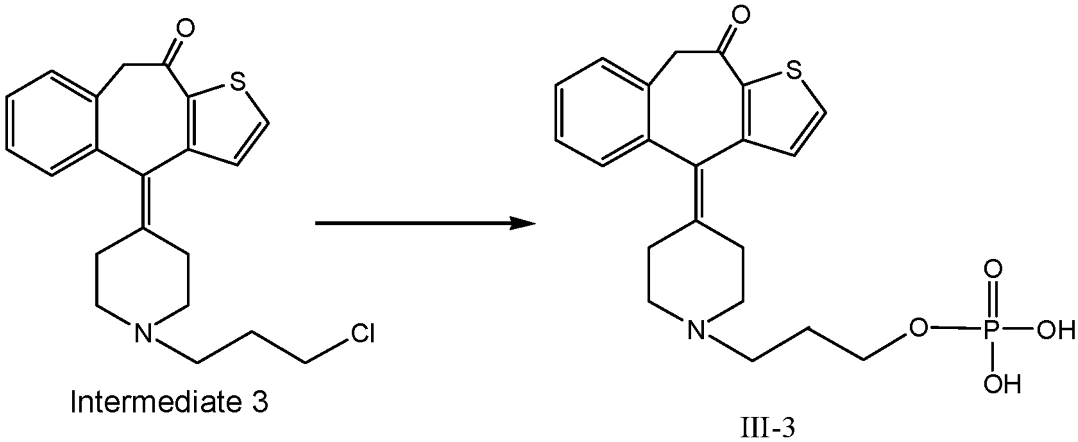

Intermediate 3

III-3

Compound III-3 was prepared using the method for preparing Intermediate III-1 in Example 14, with Intermediate 1 replaced with Intermediate 3, and other materials remained unchanged.

Example 17: Preparation of Compound III-4

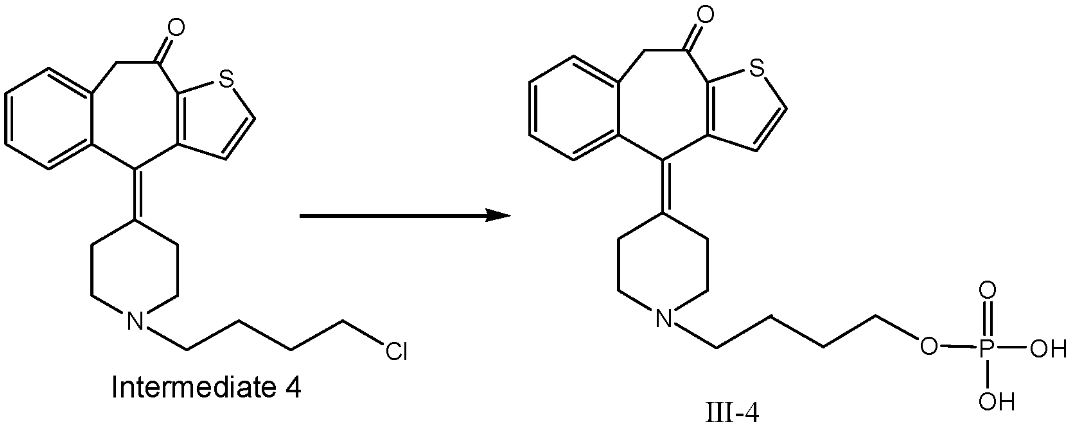

Intermediate 4

III-4

Compound III-4 was prepared using the method for preparing Intermediate III-1 in Example 14, with Intermediate 1 replaced with Intermediate 4, and other materials remained unchanged.

Example 18: Preparation of Compound III-5

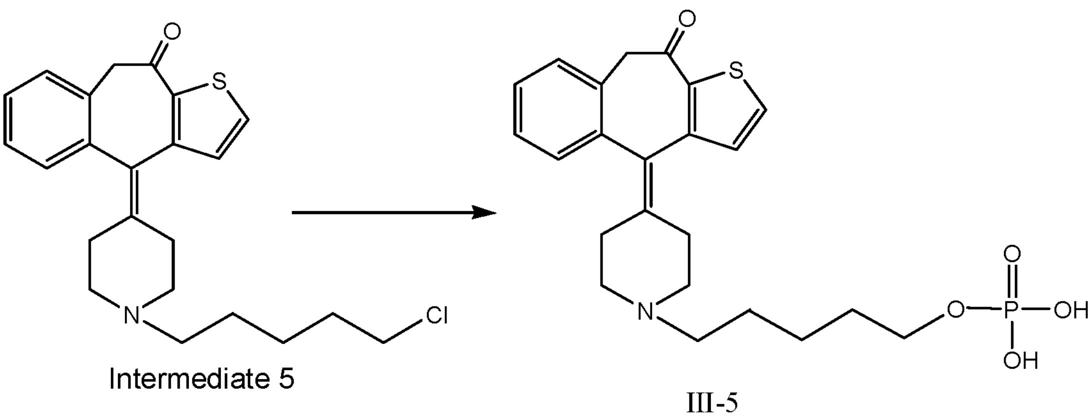

Intermediate 5

III-5

Compound III-5 was prepared using the method for preparing Intermediate III-1 in Example 14, with Intermediate 1 replaced with Intermediate 5, and other materials remained unchanged.

Example 19: Preparation of Compound III-6

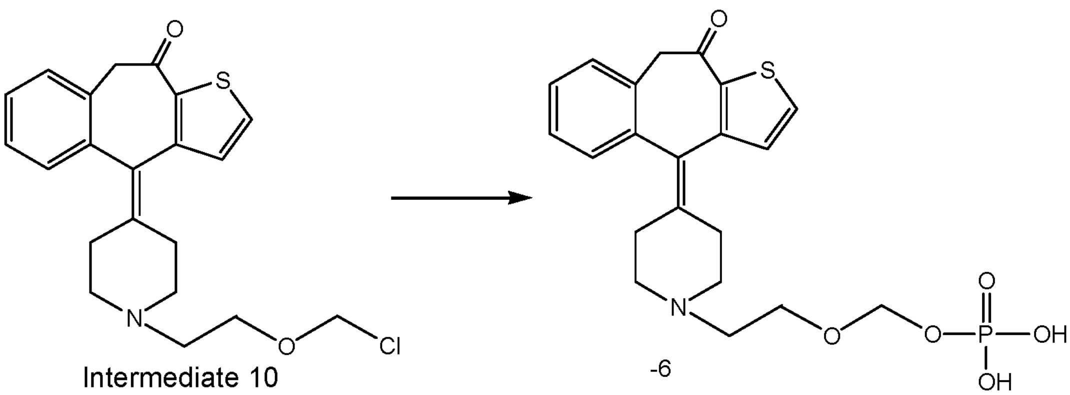

Intermediate 10

-6

Compound III-6 was prepared using the method for preparing Intermediate III-1 in Example 14, with Intermediate

1 replaced with Intermediate 10, and other materials remained unchanged.

Example 20: Preparation of Compound III-7

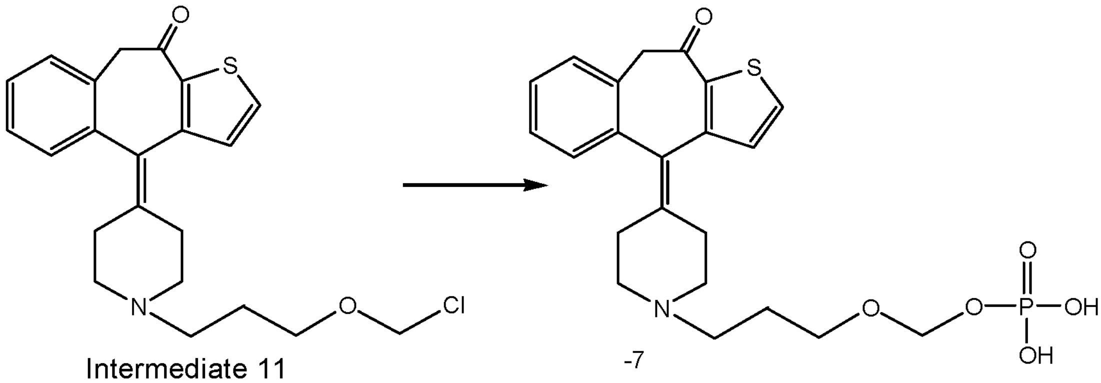

Intermediate 11

-7

Compound III-7 was prepared using the method for preparing Intermediate III-1 in Example 14, with Intermediate 1 replaced with Intermediate 11, and other materials remained unchanged.

Example 21: Preparation of Compound III-8

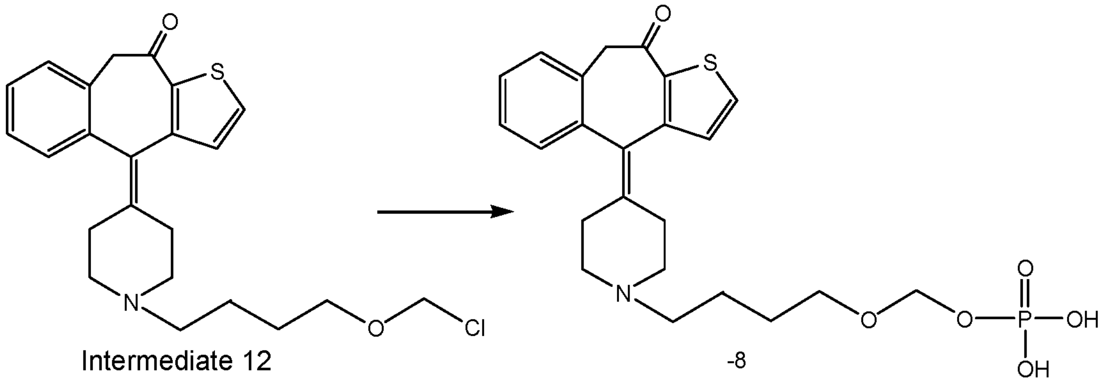

Intermediate 12

-8

Compound III-8 was prepared using the method for preparing Intermediate III-1 in Example 14, with Intermediate 1 replaced with Intermediate 12, and other materials remained unchanged.

Example 22: Preparation of Compound III-9

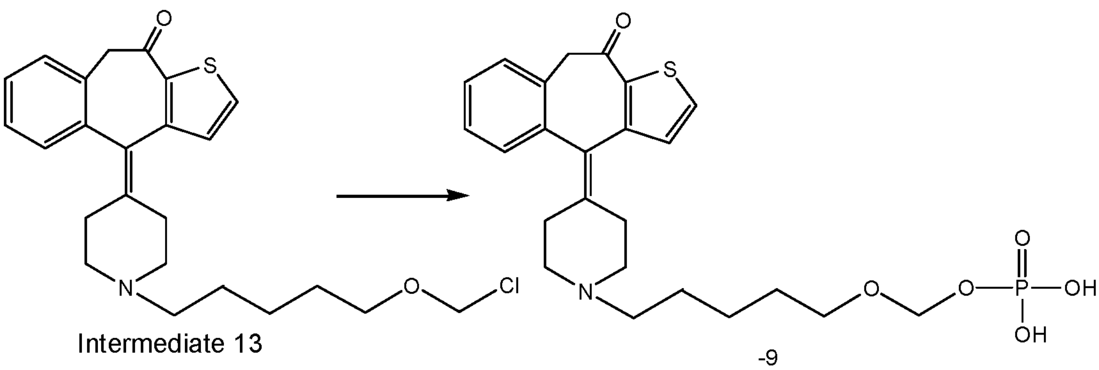

Intermediate 13

-9

Compound III-9 was prepared using the method for preparing Intermediate III-1 in Example 14, with Intermediate 1 replaced with Intermediate 13, and other materials remained unchanged.

Example 23: Preparation of Compound IV-1

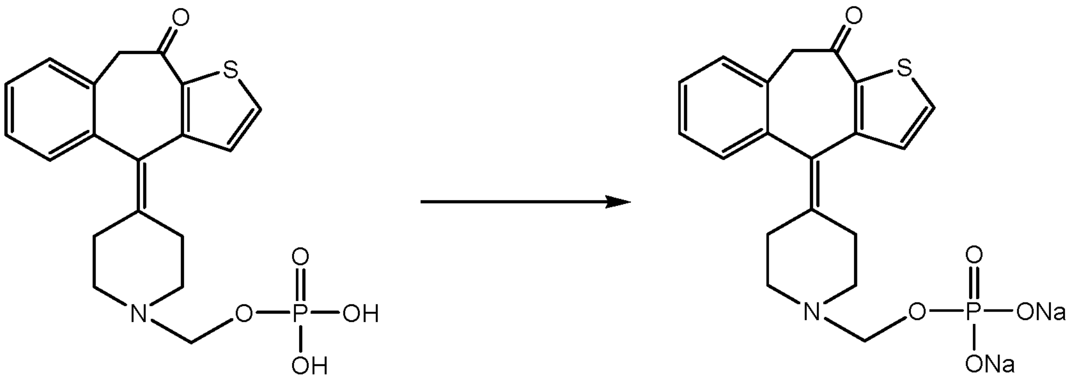

III-1

-1

To a reaction flask was added 10 g of Compound III-1 and then 50ml of ethanol added and the resulting mixture was heated to 50°C and stirred to dissolve the solids. 1.97g of sodium hydroxide added and stirred to dissolve, stirring was lasted for 20 minutes when the temperature was held. The resulting mixture was filtered, the filtrate was cooled to 0-5°C, and crystallization was conducted for 4 hours when the temperature was kept. Filtration was followed and the filter cake was dried by forced air blow to obtain 9.5g of Compound IV-1 with a yield of 85.7%.

Example 24: Preparation of Compound IV-2

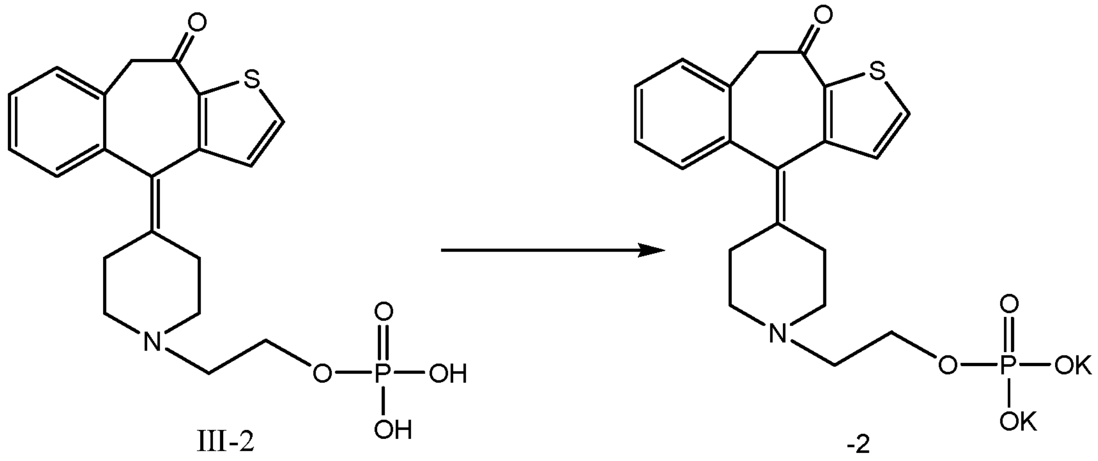

III-2 -2

Compound IV-2 was prepared using the method for preparing Compound IV-1 in Example 23, with Compound III-1 replaced with Compound III-2, sodium hydroxide replaced with potassium hydroxide, and other materials remained unchanged.

Example 25: Preparation of Compound IV-3

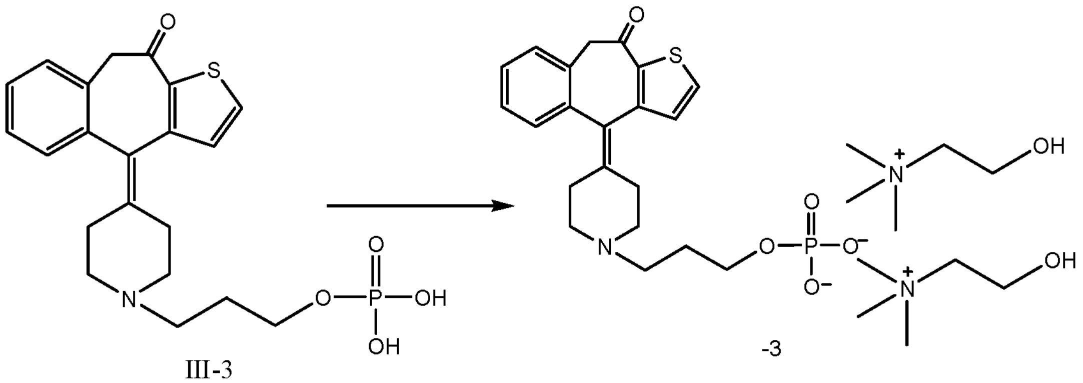

III-3 -3

Compound IV-3 was prepared using the method for preparing Compound IV-1 in Example 23, with Compound III-1 replaced with Compound III-3, sodium hydroxide replaced with choline hydroxide, and other materials remained unchanged.

Example 26: Preparation of Compound IV-4

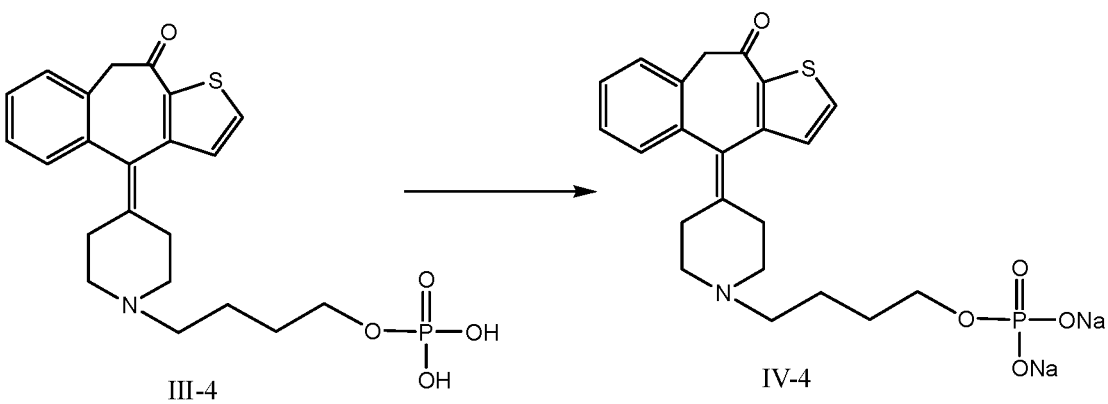

III-4 IV-4

Compound IV-4 was prepared using the method for preparing Compound IV-1 in Example 23, with Compound III-1 replaced with Compound III-4, and other materials remained unchanged.

Example 27: Preparation of Compound IV-5

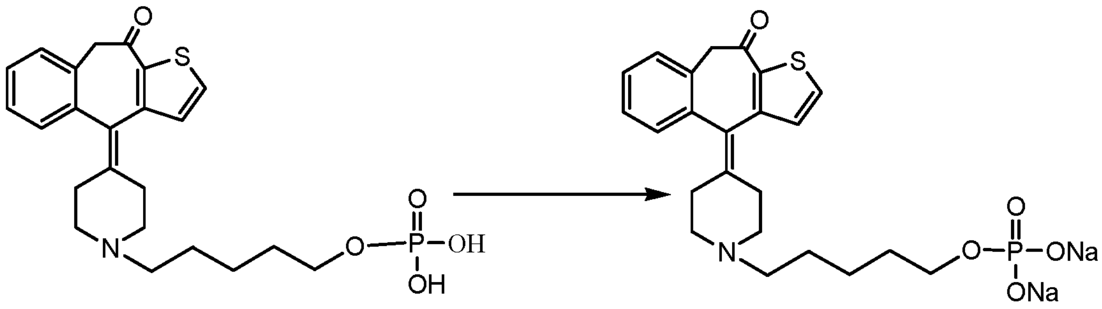

III-5 IV-5

Compound IV-5 was prepared using the method for preparing Compound IV-1 in Example 23, with Compound III-1 replaced with Compound III-5, and other materials remained unchanged.

Example 28: Preparation of Compound IV-6

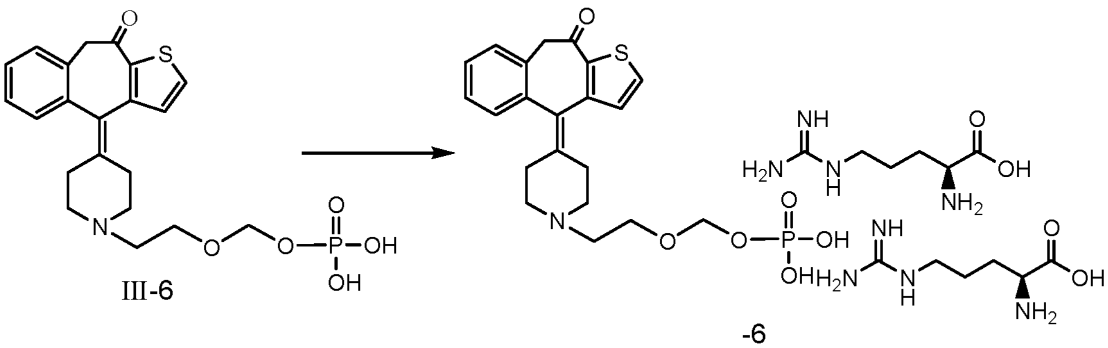

III-6

-6

Compound IV-6 was prepared using the method for preparing Compound IV-1 in Example 23, with Compound III-1 replaced with Compound III-6, sodium hydroxide replaced with arginine, and other materials remained unchanged.

Example 29: Preparation of Compound IV-7

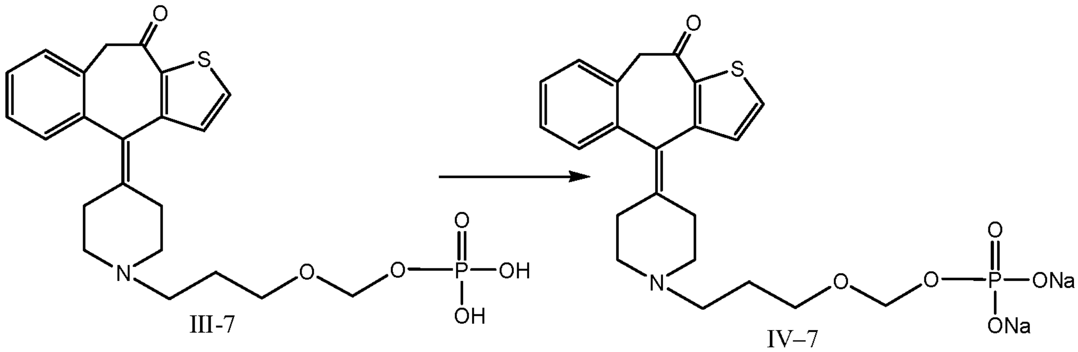

III-7 IV-7

Compound IV-7 was prepared using the method for preparing Compound IV-1 in Example 23, with Compound III-1 replaced with Compound III-7, and other materials remained unchanged.

Example 30: Preparation of Compound IV-8

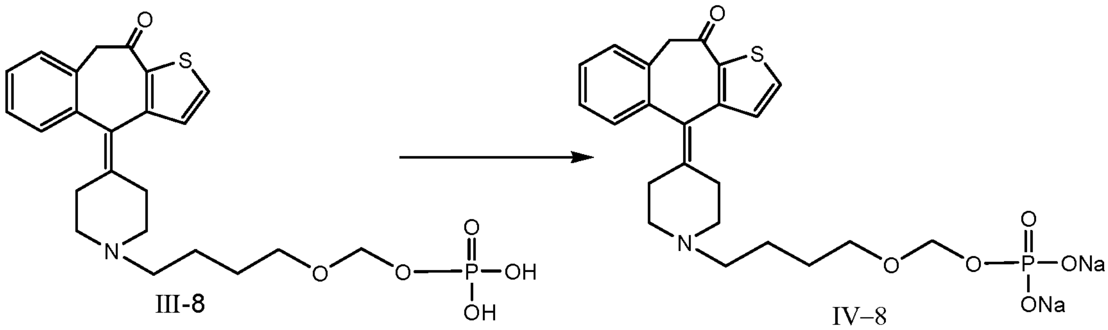

III-8 IV-8

Compound IV-8 was prepared using the method for preparing Compound IV-1 in Example 23, with Compound III-1 replaced with Compound III-8, and other materials remained unchanged.

Example 31: Preparation of Compound IV-9

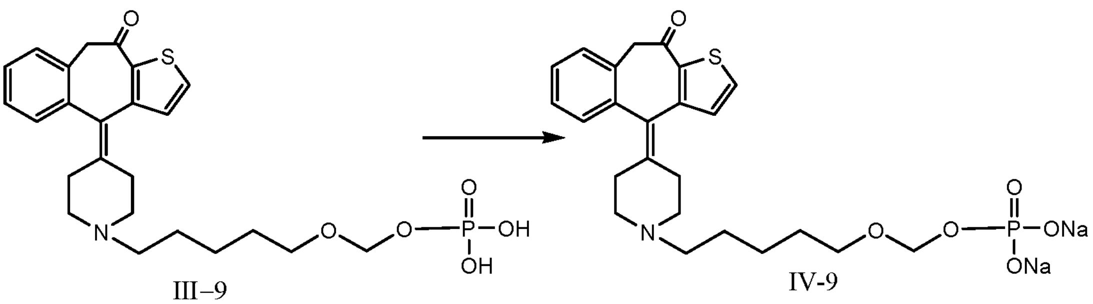

III-9 IV-9

Compound IV-9 was prepared using the method for preparing Compound IV-1 in Example 23, with Compound III-1 replaced with Compound III-9, and other materials remained unchanged.

Investigation of physical and chemical properties

Example 32: Investigation of the solubility of the compounds of the present disclosure

The solubility in water, methanol, and isopropanol of the compounds of the present disclosure and the comparative compounds were measured separately.

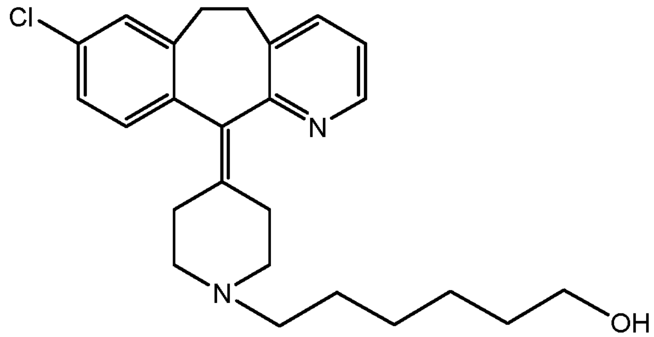

Comparative compound 1: hydroxyhexyl desloratadine

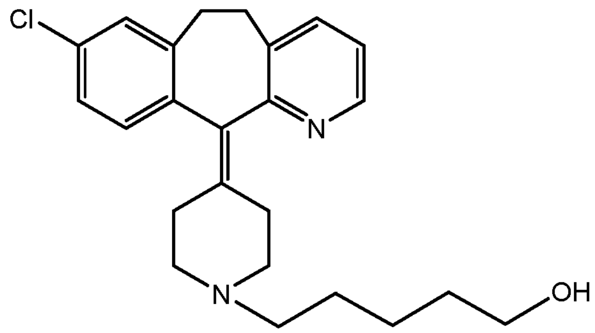

Comparative compound 2: hydroxypentyl desloratadine

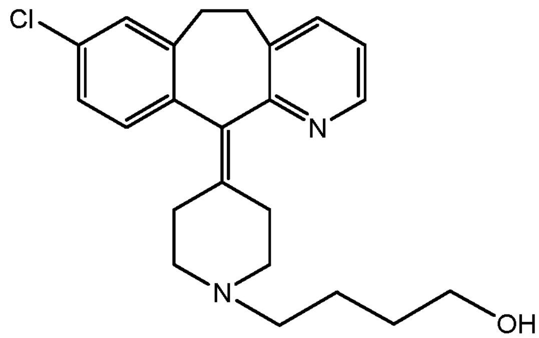

Comparative compound 3: hydroxybutyl desloratadine

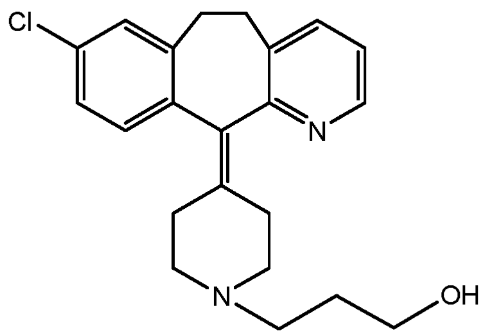

Comparative compound 4: hydroxypropyl desloratadine

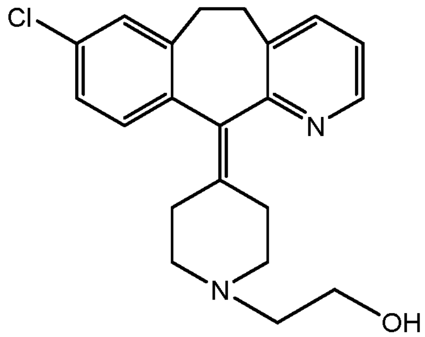

Comparative compound 5: hydroxyethyl desloratadine

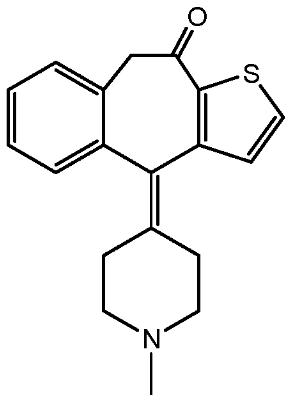

Comparative compound 6: ketotifen

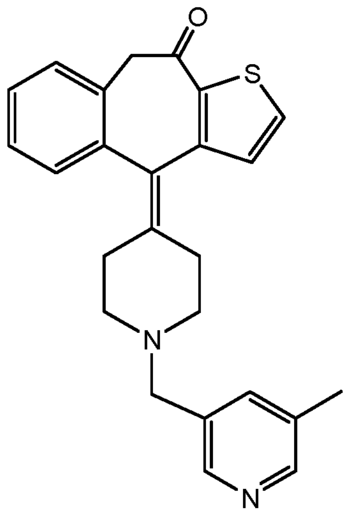

Comparative compound 7: rupatifen.

The test results are shown in Table 1 below.

Table 1: Results for solubility test

| Compound | Water | Methanol | Isopropanol |
|---|---|---|---|
| III-1 | 20mg/ml | 35mg/ml | 24mg/ml |
| III-2 | 20mg/ml | 35mg/ml | 24mg/ml |

(continued)

| Compound | Water | Methanol | Isopropanol |
|---|---|---|---|
| III-3 | 20mg/ml | 35mg/ml | 24mg/ml |
| III-4 | 20mg/ml | 35mg/ml | 24mg/ml |
| III-5 | 20mg/ml | 38mg/ml | 24mg/ml |
| III-6 | 20mg/ml | 38mg/ml | 24mg/ml |
| III-7 | 20mg/ml | 38mg/ml | 24mg/ml |
| III-8 | 20mg/ml | 38mg/ml | 24mg/ml |
| III-9 | 20mg/ml | 38mg/ml | 24mg/ml |
| IV-1 | 488mg/ml | 35mg/ml | 1mg/ml |
| IV-2 | 348mg/ml | 35mg/ml | 1mg/ml |
| IV-3 | 404m/ml | 38mg/ml | 1mg/ml |
| IV-4 | 476m/ml | 35mg/ml | 1mg/ml |
| IV-5 | 478mg/ml | 35mg/ml | 1mg/ml |
| IV-6 | 289mg/ml | 28mg/ml | 2mg/ml |
| IV-7 | 466mg/ml | 35mg/ml | 2mg/ml |
| IV-8 | 470mg/ml | 35mg/ml | 2mg/ml |
| IV-9 | 473mg/ml | 35mg/ml | 2mg/ml |
| Ketotifen | Practically insoluble | 30mg/ml | 20mg/ml |
| Rupatifen | Practically insoluble | 30mg/ml | 20mg/ml |
| Hydroxyethyl desloratadine | Practically insoluble | 27mg/ml | 21mg/ml |
| Hydroxypropyl desloratadine | Practically insoluble | 24mg/ml | 26mg/ml |
| Hydroxybutyl desloratadine | Practically insoluble | 22mg/ml | 28mg/ml |
| Hydroxypentyl desloratadine | Practically insoluble | 19mg/ml | 29mg/ml |
| Hydroxyhexyl desloratadine | Practically insoluble | 15mg/ml | 32mg/ml |

The results show that the solubility of the compounds of the present invention in water was better than that of ketotifen, rupatifen, and hydroxyethyl desloratadine-based compounds. In particular, after being formulated into a sodium salt, the solubility in water became higher, and it was more suitable for the preparation of intravenous formulations, and the solubility of the compounds of the present invention in methanol and isopropanol was comparable to that of the comparative compounds.

Efficacy studies

Example 33: Antiasthmatic effect of the present disclosure compound on histamine-induced asthma in guinea pigs

The guinea pigs were placed in a polymethyl methacrylate bell jar and a histamine hydrochloride solution was sprayed ultrasonically (0.8 mg/mL) for 40 seconds. The time point at which the guinea pigs developed asthma was recorded. The time of convulsion and fall was taken as the latency period. Guinea pigs with a latency period exceeding 180 seconds were not selected. The selected 125 guinea pigs were randomly divided into 25 groups, with 5 guinea pigs in each group: the normal control group, the ketotifen group (1 mg/kg), the rupatifen group (1 mg/kg), the hydroxyethyl desloratadine group (1 mg/kg), the hydroxypropyl desloratadine group (1 mg/kg), the hydroxybutyl desloratadine group (1 mg/kg), the hydroxypentyl desloratadine group (1 mg/kg), the hydroxyhexyl desloratadine group (1 mg/kg), and the present-disclosure compound group (1 mg/kg). The guinea pigs were administered by oral gavage at a dose of 2 mL/kg·bw. Sixty minutes after the administration, the guinea pigs were placed in bell jars, and histamine hydrochloride solution was sprayed under the same conditions as in the previous selection. The latency period for the onset of asthma was recorded. If asthma did not occur within 8 minutes, it was counted as 8 minutes. The results were processed statistically.

The results are shown in Table 2.

Table 2 Results for the antagonistic effect of the test compounds against histamine ( $\overline{X}\pm s$, n = 5)

| Group | Pre-administration latency period (s) | Post-administration latency period (s) |
|---|---|---|
| Normal control group | 98.55±45.25 | 108.43±46.22 |
| Ketotifen group | 98.79±51.72 | 263.11±54.90 |
| Rupatifen group | 99.10±52.01 | 253.23±52.29 |
| Hydroxyethyl desloratadine group | 94.24±47.71 | 281.12±54.82 |
| Hydroxypropyl desloratadine group | 96.83±50.21 | 285.28±56.32 |
| Hydroxybutyl desloratadine group | 99.18±51.15 | 286.23±55.29 |
| Hydroxypentyl desloratadine group | 98.05±50.25 | 284.29±57.34 |
| Hydroxyhexyl desloratadine group | 98.86±51.74 | 281.18±53.76 |
| Compound III-1 | 98.57±49.10 | 376.25±51.77 |
| Compound III-2 | 99.48±50.88 | 377.22±50.87 |
| Compound III-3 | 100.17±47.38 | 368.01±50.67 |
| Compound III-4 | 99.07±50.25 | 369.49±51.06 |
| Compound III-5 | 102.32±54.23 | 375.52±49.26 |
| Compound III-6 | 98.68±49.22 | 370.03±52.11 |
| Compound III-7 | 102.04±48.89 | 377.23±50.23 |
| Compound III-8 | 99.49±51.11 | 376.24±50.42 |
| Compound III-9 | 100.10±47.83 | 267.95±51.16 |
| Compound IV-1 | 98.45±51.22 | 368.83±49.03 |
| Compound IV-2 | 100.11±50.26 | 380.27±50.25 |
| Compound IV-3 | 99.10±50.25 | 371.38±49.10 |
| Compound IV-4 | 97.88±50.01 | 376.89±51.43 |
| Compound IV-5 | 96.25±47.12 | 377.11±50.43 |
| Compound IV-6 | 98.46±48.23 | 370.56±50.81 |
| Compound IV-7 | 100.51:1:45.27 | 373.33±52.76 |
| Compound IV-8 | 99.00±45.99 | 370.23±50.29 |
| Compound IV-9 | 98.68±48.06 | 369.11±49.40 |

The test results show that the post-administration latency of the compounds of the present disclosure is significantly longer than that of the compounds in the normal control group, the ketotifen group, the rupatifen group, and the hydroxyethyl desloratadine-based compoud group, indicating that the compounds of the present disclosure are superior to the control groups and the normal groups in suppressing asthma.

Example 34: Effect on muscle tone of isolated ileal smooth muscle of guinea pigs

The guinea pig was knocked unconscious with a wooden stick, and the abdomen was immediately dissected to separate the ileum about 15 cm in length. The contents of the intestinal segment were flushed with Tyrode's solution and placed in Tyrode's solution at a constant temperature of 37°C for later use, and oxygen was supplied simultaneously. The experimental intestinal tract (1 cm in length) was cut and placed into 20 ml of Tyrode's solution at a constant temperature of 37°C with a continuous oxygen supply. One end of the intestine was fixed on the ventilation hook and the other end was connected to the muscle tone transducer and led to the computer interface. The muscle tone value of the ileal smooth muscle was recorded with a BL system. The experiment was carried out after the intestinal segment contraction became stable.

After the ileum contraction curve stabilized, the tone values before adding the drug were recorded, then the drug or DMSO was added, and the average tone value of the ileum contraction curve at that point was recorded 5 minutes later. The operation was repeated 8 times. The antispasmodic percentage was calculated according to the following formula.

$$\text{Antispasmodic percentage} = \frac{\text{Tone value before adding subject compound} - \text{Tone value after adding subject compound}}{\text{Tone value before adding subject compound}} \times 100\%$$

The results are shown in Table 3.

Table 3. Results for the antagonistic effect of the test compounds against histamine ( $\overline{X}\pm s$, n = 8)

| Group | Smooth muscle tone (g) | | Antispasmodic percentage (%) |
|---|---|---|---|
| | Before administration | After administration | |
| Control group | 1.71±0.18 | 1.69±0.14 | 1.17±7.05 |
| Ketotifen group | 1.70±0.13 | 1.30±0.30 | 23.53±10.10 |
| Rupatifen group | 1.72±0.11 | 1.31±0.25 | 23.84±11.10 |
| Hydroxyethyl desloratadine group | 1.70±0.15 | 1.33±0.27 | 21.76±10.04 |
| Hydroxypropyl desloratadine group | 1.71±0.13 | 1.30±0.24 | 23.97±11.12 |
| Hydroxybutyl desloratadine group | 1.73±0.16 | 1.31±0.28 | 24.27±12.31 |
| Hydroxypentyl desloratadine group | 1.71±0.18 | 1.28±0.24 | 25.14±12.21 |
| Hydroxyhexyl desloratadine group | 1.72±0.16 | 1.30±0.30 | 24.42±11.13 |
| Compound III-1 | 1.70±0.17 | 1.18±0.25 | 30.59±18.11 |
| Compound III-2 | 1.70±0.21 | 1.21±0.24 | 28.82±16.05 |
| Compound III-3 | 1.72±0.18 | 1.20±0.24 | 30.23±17.82 |
| Compound III-4 | 1.72±0.16 | 1.16±0.23 | 32.55±18.33 |
| Compound III-5 | 1.70±0.20 | 1.13±0.31 | 33.53±19.21 |
| Compound III-6 | 1.71±0.24 | 1.14±0.30 | 33.33±19.04 |
| Compound III-7 | 1.72±0.22 | 1.20±0.25 | 30.23±19.04 |
| Compound III-8 | 1.70±0.29 | 1.16±0.21 | 31.76±18.69 |
| Compound III-9 | 1.71±0.3 0 | 1.12±0.29 | 34.50±19.89 |
| Compound IV-1 | 1.72±0.18 | 1.14±0.31 | 33.72±18.32 |
| Compound IV-2 | 1.71±0.18 | 1.19±0.31 | 30.40±18.50 |
| Compound IV-3 | 1.68±0.33 | 1.16±0.29 | 30.95±19.69 |
| Compound IV-4 | 1.70±0.28 | 1.18±0.26 | 30.59±19.00 |
| Compound IV-5 | 1.72±0.26 | 1.14±0.30 | 33.72±19.05 |
| Compound IV-6 | 1.70±0.25 | 1.17±0.28 | 31.17±19.54 |
| Compound IV-7 | 1.73±0.31 | 1.15±0.25 | 33.52±19.74 |
| Compound IV-8 | 1.70±0.35 | 1.14+0.31 | 32.94±19.45 |
| Compound IV-9 | 1.73±0.30 | 1.16±0.28 | 32.95±19.04 |

After the ileal peristalsis curve stabilized, the tone values before adding the compound were recorded, and then 0.05 mL of histamine was added. When the maximum contraction was reached, 0.05 mL of different compounds or DMSO

were added, respectively, and the average tone value when adding histamine and 3 minutes after adding the compound were observed and recorded. The operation was repeated 8 times. The antispasmodic percentage was calculated using the following equation.

$$\text{Antispasmodic percentage} = \frac{\text{Tone value after adding histamine} - \text{Tone value after adding subject compound}}{\text{Tone value after adding histamine}} \times 100\%$$

The results are shown in Table 4

Table 4. Results for the antagonistic effect of the test compounds against histamine ( x±s, n = 8)

| Group | Smooth muscle tone (g) | | | Antispasmodic percentage (%) |
|---|---|---|---|---|
| | Before administration | After addition of histamine | After administration | |
| Control group | 1.76±0.19 | 3.40±0.97 | 3.37±0.98 | 0.88±3.11 |
| Ketotifen group | 1.76±0.16 | 3.41±0.89 | 1.50±0.48 | 56.01±12.18 |
| Rupatifen group | 1.74±0.18 | 3.41±0.94 | 1.52±0.46 | 55.42±11.38 |
| Hydroxyethyl desloratadine group | 1.76±0.16 | 3.40±0.86 | 1.41±0.43 | 58.53±10.79 |
| hydroxypropyl desloratadine group | 1.72±0.15 | 3.40±0.95 | 1.46±0.49 | 57.05±12.07 |
| Hydroxybutyl desloratadine group | 1.72±0.16 | 3.41±0.90 | 1.45±0.47 | 57.77±13.69 |
| Hydroxypentyl desloratadine group | 1.75±0.19 | 3.42±0.92 | 1.44±0.51 | 57.48±12.05 |
| Hydroxyhexyl desloratadine group | 1.75±0.16 | 3.41±0.90 | 1.43±0.47 | 58.06±13.39 |
| Compound III-1 | 1.79±0.21 | 3.43±1.06 | 1.14±0.23 | 66.76±16.11 |
| Compound III-2 | 1.75±0.18 | 3.41±0.89 | 1.18±0.21 | 65.39±17.28 |
| Compound III-3 | 1.77±0.20 | 3.42±1.00 | 1.14±0.21 | 66.66±15.49 |
| Compound III-4 | 1.78±0.17 | 3.44±0.96 | 1.23±0.24 | 64.24±17.11 |
| Compound III-5 | 1.73±0.21 | 3.43±0.93 | 1.20±0.21 | 65.01±18.17 |
| Compound III-6 | 1.78±0.20 | 3.43±0.92 | 1.22±0.23 | 64.43±17.18 |
| Compound III-7 | 1.76±0.17 | 3.44±0.86 | 1.16±0.22 | 66.28±16.16 |
| Compound III-8 | 1.78±0.20 | 3.42±0.99 | 1.12±0.21 | 66.47±18.83 |
| Compound III-9 | 1.74±0.17 | 3.45±0.89 | 1.17±0.24 | 67.25±17.74 |
| Compound IV-1 | 1.75±0.19 | 3.50±1.10 | 1.18±0.24 | 66.28±18.26 |
| Compound IV-2 | 1.74±0.15 | 3.45±0.95 | 1.14±0.23 | 66.95±17.07 |
| Compound IV-3 | 1.82±0.16 | 3.42±0.88 | 1.20±0.24 | 64.91±18.92 |
| Compound IV-4 | 1.78±0.17 | 3.44±0.98 | 1.19±0.21 | 65.40±19.07 |
| Compound IV-5 | 1.73±0.19 | 3.49±0.99 | 1.20±0.22 | 65.61±19.34 |
| Compound IV-6 | 1.76±0.17 | 3.44±0.86 | 1.15±0.20 | 66.56±19.45 |
| Compound IV-7 | 1.78±0.20 | 3.44±0.98 | 1.17±0.22 | 65.99±19.08 |
| Compound IV-8 | 1.74±0.21 | 3.43±0.97 | 1.20±0.25 | 65.01±18.56 |
| Compound IV-9 | 1.78±0.19 | 3.44±0.93 | 1.15±0.26 | 66.57±18.66 |

The test results show that the compounds of the present disclosure had higher antihistamine activity compared with those in the ketotifen group, the rupatifen group, and the hydroxyethyl desloratadine-based compound group.

Preparation of formulations

Example 35: Preparation of oral solution

Formulation:

| | |
|---|---|
| Compound IV-2 | 0.6g |
| Sucrose | 300g |
| Citric acid | 3.0g |
| Ethyl 4-hydroxybenzoate | 5ml |
| Water for injection made up to | 1000ml |

Preparation method: into 500ml of water for injection was added sucrose and the resulting mixture was stirred to full dissolution. Then 0.6g of Compound IV-2 was added to the solution and stirred until dissolution, then citric acid and ethyl 4-hydroxybenzoate were added to the solution and stirred to complete dissolution. Then water was added to make up to 1000ml, the resulting solution was filtered, divided into portions, and sterilized at 105°C for 30 minutes to obtain the product.

Example 36: Preparation of lyophilized formulation

Formulation:

| | |
|---|---|
| Compound IV-4 | 4.0g |
| Glucose | 50g |
| Hydrochloride | Adjust pH to 8-9 |
| Water for injection made up to | 1000ml |

Preparation method: To 700ml of water for injection was added Compound IV-4 and stirred to complete dissolution, then glucose was added to the solution and stirred to dissolve, the pH of the solution was adjusted to 8-9 with hydrochloric acid, filtered, and poured into 7-ml vials The vials were partially stoppered, placed in a freeze dryer, freeze dried, and the stopper was fully pressed, and the vials were capped by a capping machine.

Example 37: Preparation of tablets

Formulation:

| | |
|---|---|
| Compound III-2 | 8.0g |
| Starch | 600g |
| Microcrystalline cellulose | 200g |
| 4% Povidone K30 | Quantum Sufficit |
| Magnesium stearate | 4g |
| Formulated into | 1000 tablets |

Preparation method: prescribed amounts of starch, microcrystalline cellulose, and Compound III-2 were mixed

evenly; the materials were made into a soft material using a 4% povidone K30 solution, and a 20-mesh screen was used for granulation. The granules were dried at 40-50°C until the moisture content met requirements, and the granules were passed through a 20-mesh screen to granulate, a prescribed amount of magnesium stearate was added and finally mixed. The intermediate content was measured, and the tablet weight and determined; tablet pressing was followed.

Stability study

Example 38: Stability study of the present disclosure compound

The compound of the present disclosure was placed at a temperature of 40°C ± 2°C and a relative humidity of 75% ± 5% for 6 months, and samples were taken to determine for the descriptions, related substances, and contents. The results are shown in Table 5 below.

Table 5: Results for accelerated stability test of the present disclosure compounds

| Time | Test item | Compound III-3 | Compound III-7 | Compound IV-1 | Compound IV-2 | Compound IV-3 | Compound IV-4 | Compound IV-6 | Compound IV-8 |
|---|---|---|---|---|---|---|---|---|---|
| Month 0 | Description | White powder | White powder | White powder | White powder | White powder | White powder | White powder | White powder |
| | Related substances | 0.03% | 0.03% | 0.03% | 0.03% | 0.03% | 0.03% | 0.03% | 0.03% |
| | Content | 99.7% | 99.7% | 99.7% | 99.7% | 99.7% | 99.7% | 99.7% | 99.7% |
| Month 1 | Description | White powder | White powder | White powder | White powder | White powder | White powder | White powder | White powder |
| | Related substances | 0.05% | 0.05% | 0.05% | 0.04% | 0.05% | 0.05% | 0.05% | 0.04% |
| | Content | 99.7% | 99.7% | 99.7% | 99.7% | 99.7% | 99.7% | 99.7% | 99.7% |
| Month 2 | Description | White powder | White powder | White powder | White powder | White powder | White powder | White powder | White powder |
| | Related substances | 0.10% | 0.10% | 0.09% | 0.04% | 0.09% | 0.09% | 0.09% | 0.05% |
| | Content | 99.6% | 99.6% | 99.6% | 99.7% | 99.6% | 99.6% | 99.6% | 99.7% |
| Month 3 | Description | White powder | White powder | White powder | White powder | White powder | White powder | White powder | White powder |
| | Related substances | 0.15% | 0.15% | 0.12% | 0.06% | 0.12% | 0.12% | 0.12% | 0.06% |
| | Content | 99.5% | 99.5% | 99.6% | 99.6% | 99.6% | 99.6% | 99.6% | 99.6% |
| Month 6 | Description | White powder | White powder | White powder | White powder | White powder | White powder | White powder | White powder |
| | Related substances | 0.34% | 0.34% | 0.24% | 0.09% | 0.25% | 0.25% | 0.25% | 0.09% |
| | Content | 99.2% | 99.2% | 99.4% | 99.6% | 99.4% | 99.4% | 99.4% | 99.6% |

The results show that the present disclosure compounds had good stability under accelerated conditions. In particular the sodium salt compound had significantly better stability than other compounds.

## Claims

1. An antihistaminic compound and a pharmaceutically acceptable salt thereof, having the structure shown in Formula I:

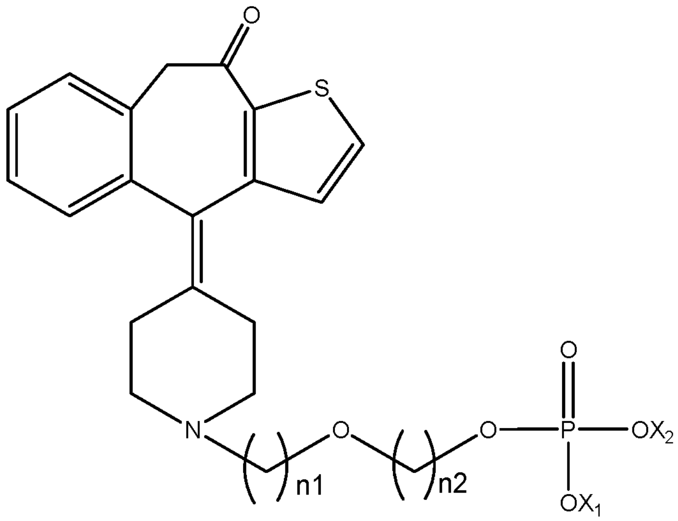

I

wherein $X_1$ is H, an alkali metal, an amino acid, meglumine, choline; $X_2$ is H, an alkali metal, an amino acid, meglumine, choline, n1 is an integer of 1-5, and n2 is an integer of 1-3.

2. The antihistaminic compound and pharmaceutically acceptable salt thereof according to claim 1, wherein the pharmaceutically acceptable salt comprises a salt formed by reacting the antihistaminic compound with an inorganic base or an organic base.

3. The antihistaminic compound and pharmaceutically acceptable salt thereof according to claim 2, wherein the organic base is selected from choline hydroxide, meglumine, or diisopropyl ethylamine.

4. The antihistaminic compound and pharmaceutically acceptable salt thereof according to claim 1, wherein the pharmaceutically acceptable salt comprises a metal salt or a salt formed by reacting the antihistaminic compound with a basic amino acid.

5. The antihistaminic compound and pharmaceutically acceptable salt thereof according to claim 4, wherin the metal salt is selected from an alkali metal salt and an alkaline earth metal salt; and wherein the alkali metal salt is selected from a sodium salt or a potassium salt, and the alkaline earth metal salt is selected from a calcium salt, a magnesium salt, or a barium salt, and the basic amino acid salt is selected from a lysine salt and an arginine salt.

6. The antihistaminic compound and pharmaceutically acceptable salt thereof according to claim 1, wherein the antihistaminic compound and pharmaceutically acceptable salt thereof are selected from the following compounds:

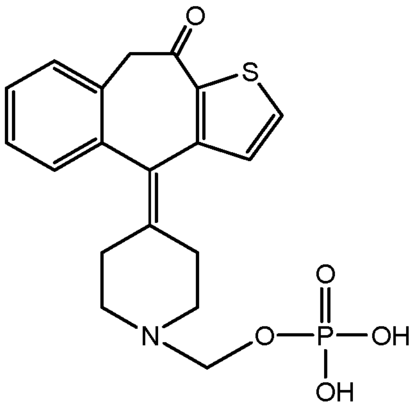

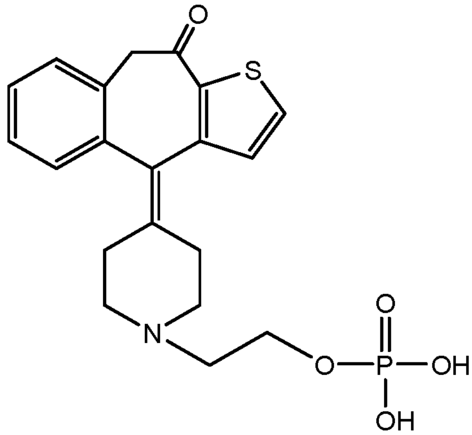

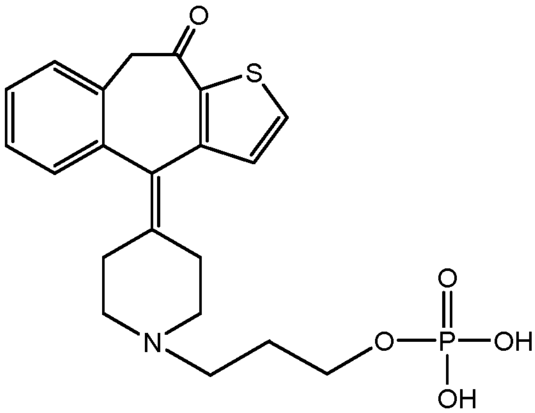

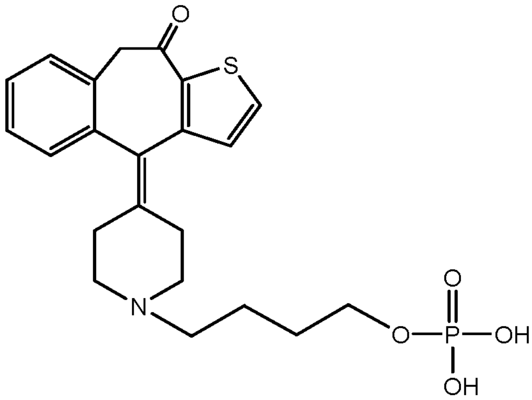

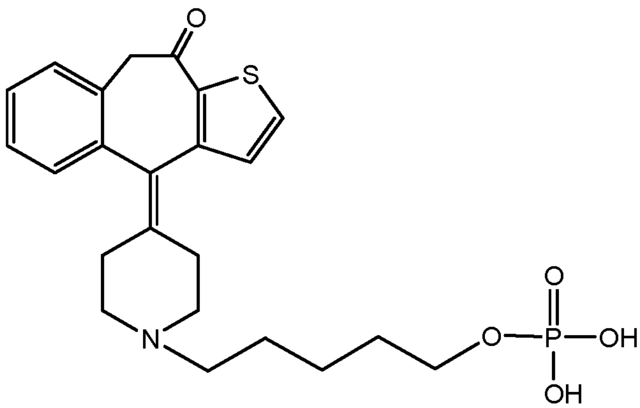

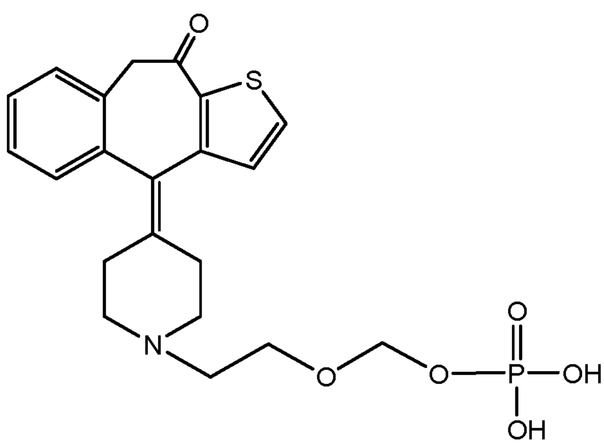

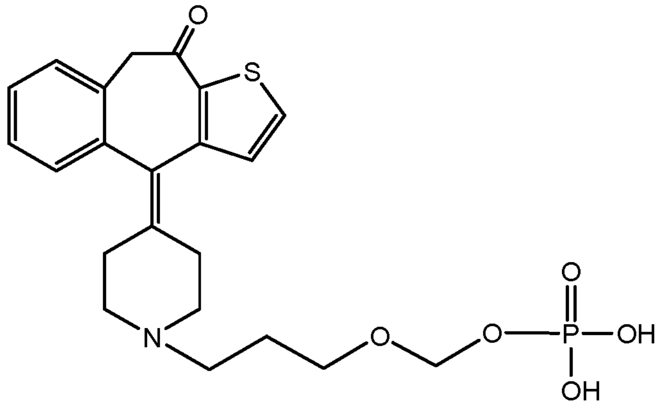

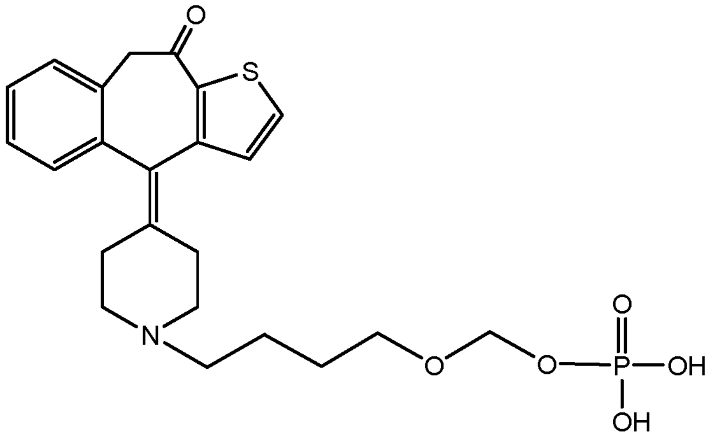

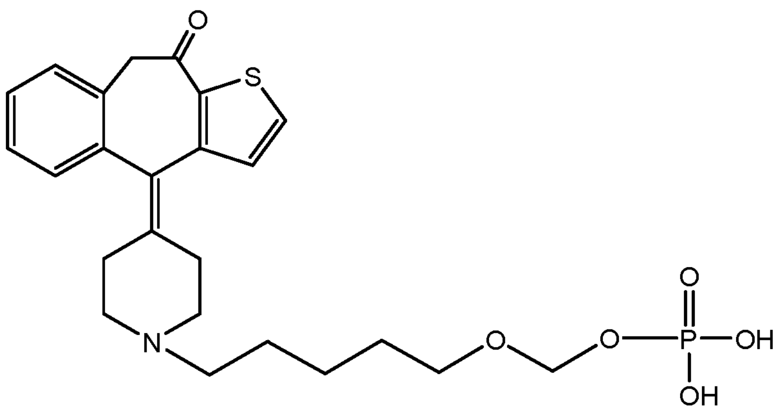

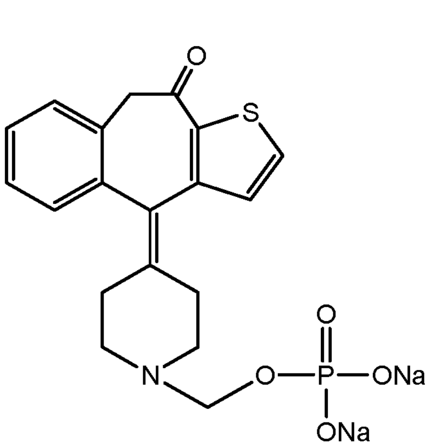

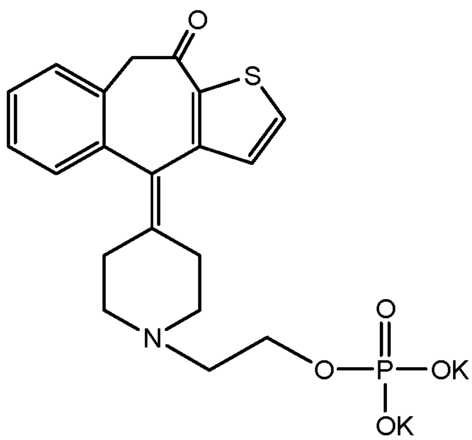

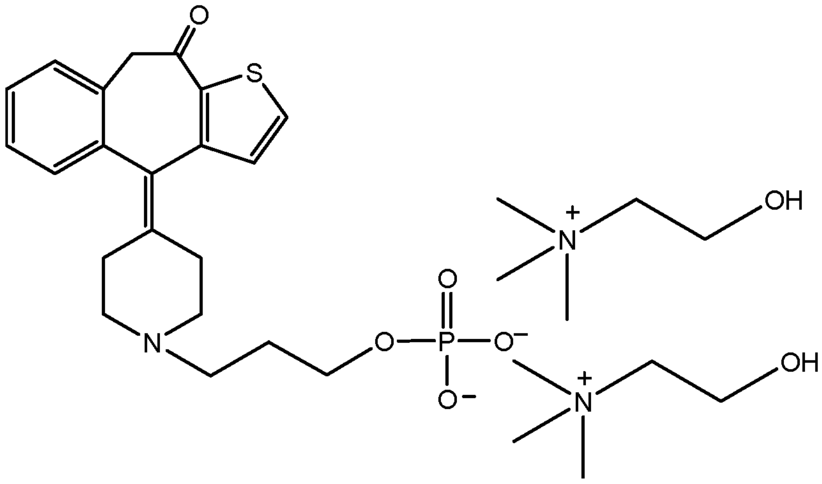

.

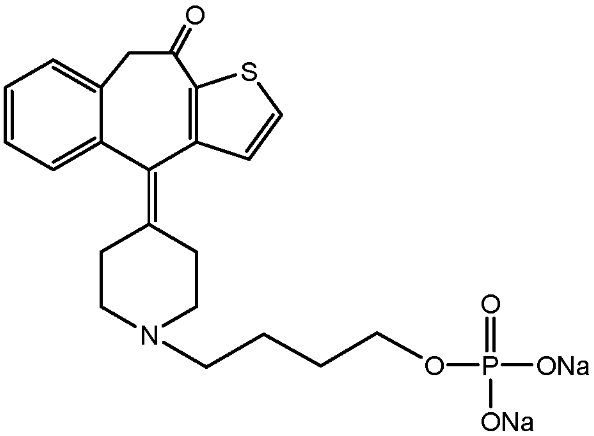

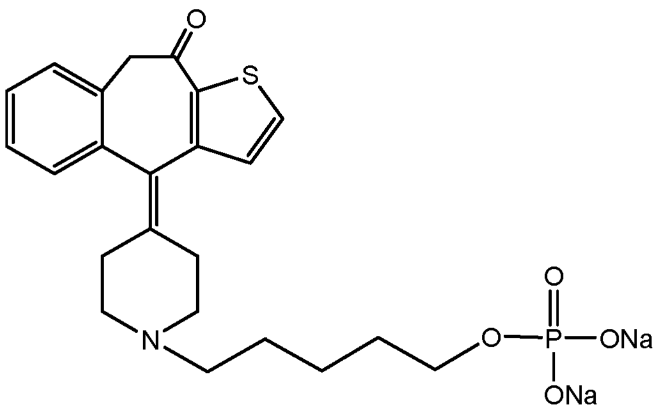

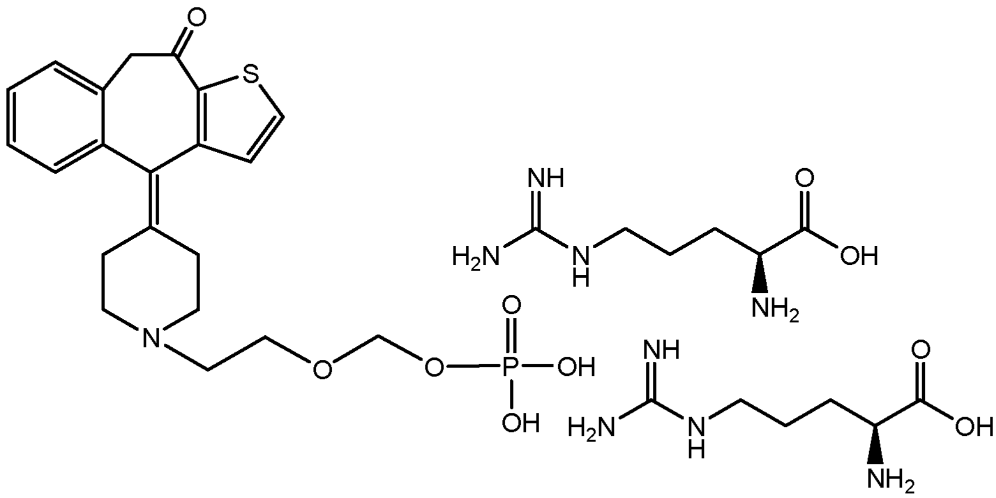

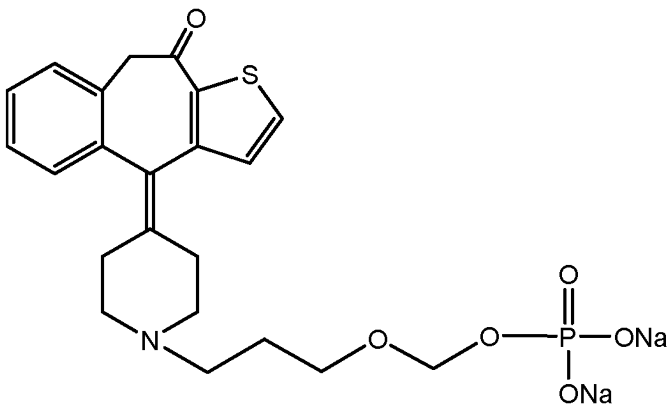

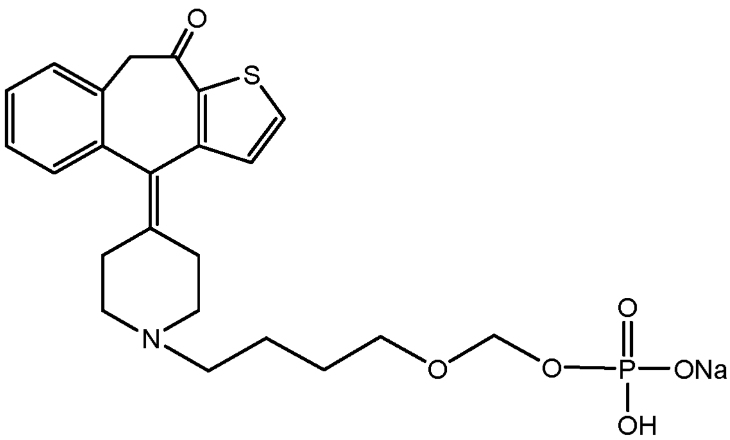

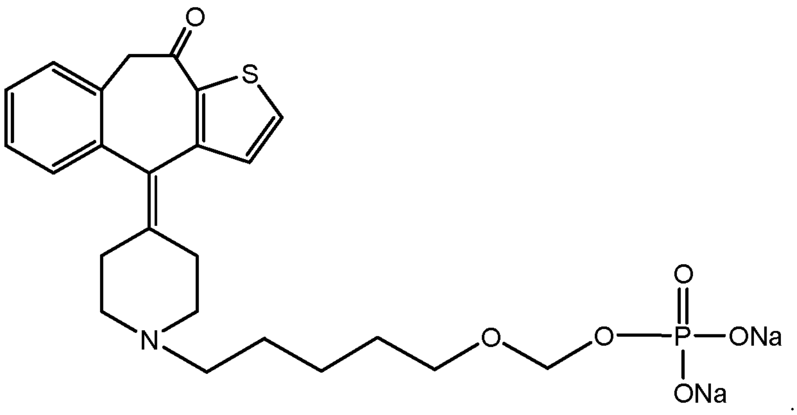

7. A pharmaceutical composition comprising a therapeutic amount of the antihistaminic compounds and/or pharmaceutically acceptable salts thereof according to any of claims 1 to 6, and a pharmaceutically acceptable excipient.

8. Use of the antihistaminic compounds or pharmaceutically acceptable salts thereof as claimed in any of claims 1 to 6 in the preparation of an antiallergic drug.

**INTERNATIONAL SEARCH REPORT**

International application No.
**PCT/CN2023/075588**

**A. CLASSIFICATION OF SUBJECT MATTER**

C07F9/6558(2006.01)i;C07C213/08(2006.01)i;C07C215/40(2006.01)i;C07C277/08(2006.01)i;A61K31/675(2006.01)i; A61P37/08(2006.01)i

According to International Patent Classification (IPC) or to both national classification and IPC

**B. FIELDS SEARCHED**

Minimum documentation searched (classification system followed by classification symbols)

C07F,C07C,A61K

Documentation searched other than minimum documentation to the extent that such documents are included in the fields searched

Electronic data base consulted during the international search (name of data base and, where practicable, search terms used)

CNABS, CNTXT, ENTXTC, VCN, CAPLUS(STN), REGISTRY(STN), CNKI, 万方, WANFANG: 抗过敏, 抗组胺, 磷酸酯, 前药, 水溶性, 杨成, 张起愿, 华创合成, allergic, antihistamine, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 1258219 A (BRIDGE PHARMA, INC.) 28 June 2000 (2000-06-28) embodiments 1-15, and, description, page 3, line 8-page 9, line 9, and page 17, line 15-page 20, line 24 | 1-8 |
| A | US 4609664 A (SANDOZ LTD.) 02 September 1986 (1986-09-02) embodiments 1-11, claims 1-11, and description, columns 7-8 | 1-8 |
| A | CN 113880801 A (HEFEI INDUSTRIAL PHARMACEUTICAL INSTITUTE CO., LTD. et al.) 04 January 2022 (2022-01-04) embodiments 1-53, and description, paragraphs 14-36 and 46-49 | 1-8 |
| A | CN 103044395 A (TIANJIN INSTITUTE OF PHARMACEUTICAL RESEARCH) 17 April 2013 (2013-04-17) description, table 2, and paragraphs 3-21 | 1-8 |

☐ Further documents are listed in the continuation of Box C. ☑ See patent family annex.

* Special categories of cited documents:
“A” document defining the general state of the art which is not considered to be of particular relevance
“D” document cited by the applicant in the international application
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **17 April 2023** | **21 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/075588**

| Patent document cited in search report | Publication date (day/month/year) | Patent family member(s) | Publication date (day/month/year) |
|---|---|---|---|
| CN 1258219 A | 28 June 2000 | PL 336007 A1 | 05 June 2000 |
| | | KR 20010005791 A | 15 January 2001 |
| | | IL 131999 A0 | 19 March 2001 |
| | | IL 131999 A | 20 June 2004 |
| | | EP 0977568 A1 | 09 February 2000 |
| | | EP 0977568 A4 | 21 March 2001 |
| | | EP 0977568 B1 | 08 March 2006 |
| | | JP 2001519789 A | 23 October 2001 |
| | | NO 994823 D0 | 04 October 1999 |
| | | NO 994823 L | 07 October 1999 |
| | | NO 311934 B1 | 18 February 2002 |
| | | RU 2193557 C2 | 27 November 2002 |
| | | HK 1027961 A1 | 02 February 2001 |
| | | ID 24487 A | 20 July 2000 |
| | | BR 9807911 A | 22 February 2000 |
| | | DE 69833748 D1 | 04 May 2006 |
| | | DE 69833748 T2 | 23 November 2006 |
| | | US 6207683 B1 | 27 March 2001 |
| | | WO 9843640 A1 | 08 October 1998 |
| | | AT 319450 T | 15 March 2006 |
| | | AU 6881298 A | 22 October 1998 |
| | | AU 733325 B2 | 10 May 2001 |
| | | NZ 337843 A | 30 November 2001 |
| | | CA 2283663 A1 | 08 October 1998 |
| | | CA 2283663 C | 25 July 2006 |
| US 4609664 A | 02 September 1986 | JPS 5777673 A | 15 May 1982 |
| | | IL 63698 A0 | 30 November 1981 |
| | | IL 63698 A | 30 June 1985 |
| | | PT 73601 A | 01 September 1981 |
| | | PT 73601 B | 28 February 1983 |
| | | FI 812635 L | 03 March 1982 |
| | | FI 75160 B | 29 January 1988 |
| | | FI 75160 C | 09 May 1988 |
| | | NZ 198220 A | 27 April 1984 |
| | | ZA 816103 B | 27 April 1983 |
| | | ZA 8106103 B | 27 April 1983 |
| | | EP 0047226 A2 | 10 March 1982 |
| | | EP 0047226 A3 | 05 May 1982 |
| | | EP 0047226 B1 | 15 May 1985 |
| | | DK 385681 A | 03 March 1982 |
| | | ES 8305714 A1 | 01 May 1983 |
| | | IE 812012 L | 02 March 1982 |
| | | IE 52182 B1 | 05 August 1987 |
| | | CA 1166245 A | 24 April 1984 |
| | | AT 13297 T | 15 June 1985 |
| | | AU 7478281 A | 11 March 1982 |
| | | AU 547111 B2 | 10 October 1985 |
| | | PH 16317 A | 05 September 1983 |
| | | DE 3170504 D1 | 20 June 1985 |
| CN 113880801 A | 04 January 2022 | None | |

Form PCT/ISA/210 (patent family annex) (July 2022)

**INTERNATIONAL SEARCH REPORT**
**Information on patent family members**

International application No.
**PCT/CN2023/075588**

| Patent document cited in search report | Publication date (day/month/year) | Patent family member(s) | Publication date (day/month/year) |
|---|---|---|---|
| CN 103044395 A | 17 April 2013 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210875404X